# EUROPÄISCHE PATENTSCHRIFT

(11) **EP 1 335 696 B1**
(45) Veröffentlichungstag und Bekanntmachung des Hinweises auf die Patenterteilung: **05.09.2007**
(21) Anmeldenummer: 01996352.9
(22) Anmeldetag: 10.11.2001
(51) Int. Cl.: A61K 8/00, A61K 8/30, A61K 8/72, A61Q 5/10, C12N 9/02, D06P 3/08

(54) **ENZYMATISCHES FÄRBEMITTEL**
ENZYMATIC COLOURING AGENTS
COLORANT ENZYMATIQUE

(30) Priorität: 20.11.2000 DE 10057547; 04.04.2001 DE 10116759
(43) Veröffentlichungstag der Anmeldung: 20.08.2003
(73) Patentinhaber: Henkel Kommanditgesellschaft auf Aktien, 40589 Düsseldorf (DE)
(72) Erfinder: KLEEN, Astrid, 40699 Erkrath (DE); FRAUENDORF, Bianca, 42549 Velbert (DE)
(86) Internationale Anmeldenummer: PCT/EP2001/013025
(87) Internationale Veröffentlichungsnummer: WO 2002/039966

(56) Entgegenhaltungen:
- EP-A- 1 013 260
- WO-A-99/20236
- FR-A- 2 769 215
- FR-A- 2 773 477
- DATABASE WPI Week 199637 Derwent Publications Ltd., London, GB; AN 1996-368055 XP002215918 & JP 08 175935 A (REAL KAGAKU), 9. Juli 1996 (1996-07-09)
- PATENT ABSTRACTS OF JAPAN vol. 2000, no. 10, 17. November 2000 (2000-11-17) & JP 2000 186020 A (LION CORP), 4. Juli 2000 (2000-07-04)

## Beschreibung

Die vorliegende Erfindung betrifft Mittel zum Färben keratinischer Fasern, die unter anderem mindestens ein Enzym, das die Oxidation der Farbstoffvorprodukte katalysieren kann, und eine Wirkstoffkombination aus mindestens einem Zuckertensid und einem Reduktionsmittel enthalten, die Verwendung dieser Mittel zum Färben keratinischer Fasern sowie verschiedene Verfahren zur Färbung keratinischer Fasern mit diesen Mitteln.

Menschliches Haar wird heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine herausragende Rolle.

Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Haar aufziehen und keinen oxidativen Prozeß zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel deutlich empfindlicher als die oxidativen Färbungen, so daß dann sehr viel schneller eine vielfach unerwünschte Nuancenverschiebung oder gar eine sichtbare "Entfärbung" eintritt.

Für dauerhafte, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich durch hervorragende, lang anhaltende Färbeergebnisse aus. Für natürlich wirkende Färbungen muß üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet.

Die oxidative Entwicklung der Färbung kann grundsätzlich mit Luftsauerstoff erfolgen. Bevorzugt wird jedoch ein chemisches Oxidationsmittel eingesetzt. Als Oxidationsmittel kommen Persulfate, Chlorite und insbesondere Wasserstoffperoxid oder dessen Anlagerungsprodukte an Harnstoff, Melamin sowie Natriumborat in Frage. Üblicherweise wird eine etwa 2-9%ige wäßrige Wasserstoffperoxidlösung eingesetzt. Unter der Einwirkung derart hoher Oxidationsmittelkonzentrationen können die keratinischen Fasern, insbesondere wenn sie bereits dauergewellt oder gebleicht sind, geschädigt werden, und in seltenen Fällen können durch diese hohen Konzentrationen auch Hautirritationen auftreten.

Ein wesentlicher Lösungsansatz zu dieser Problematik geht von der Reduzierung der Oxidationsmittelkonzentration aus. Es wurde daher in der Vergangenheit einerseits nach Farbstoffvorprodukten gesucht, die aufgrund ihrer chemischen Struktur bereits durch geringe Mengen Wasserstoffperoxid oder durch Luftsauerstoff oxidiert werden können. Andererseits wurde die Verwendung von Enzymen als Biokatalysatoren vorgeschlagen, die den erwünschten Oxidationsprozess mit sehr wenig oder ganz ohne Wasserstoffperoxid nur in der Anwesenheit von Luftsauerstoff katalysieren können.

In der deutschen Offenlegungsschrift DE-OS-21 55 390 wird ein enzymaktiviertes, oxidatives Haarfärbeverfahren beschrieben, bei dem geringe Mengen H₂O₂ in Kombination mit einer Peroxidase eingesetzt werden. Auch in der EP-A1-0 310 675 werden enzymatische Haarbehandlungsmittel offenbart, die mindestens eine Zwei-Elektronenreduzierende Oxidase, die Sauerstoff als Akzeptor nutzt, enthalten. In der EP-B1-0 548 620 werden enzymatische Haarfärbemittel beschrieben, bei denen die Oxidation der Farbstoffvorprodukte durch Einsatz einer Peroxidase katalysiert wird.

Schließlich werden in der EP-A2-0 795 313 enzymatische Haarfärbemittel beschrieben, die ein Sauerstoff-Oxidoreduktase/Substrat-System und eine Peroxidase sowie als Kupplerkomponenten zwingend ein m-Phenylendiaminderivat enthalten. Alle diese Färbemittel können aber bezüglich der erzielbaren Färbeleistung (Intensität, Nuance, Glanz, Echtheitseigenschaften) noch nicht vollständig überzeugen.

Im Rahmen der FR 2 769 215 werden enzymatische Färbemittel auf Basis von Z-Elektoren Oxido-reduizlasen offenbart.

Die Technik der leicht-oxidierbaren Farbstoffvorprodukte hat ebenso wie die bislang beschriebene enzymatische Farbentwicklung den Nachteil, daß im Vergleich zu den herkömmlichen Verfahren insbesondere bezüglich der Intensität, des Glanzes und der Echtheitseigenschaften der Färbungen schlechtere Ergebnisse erzielt werden. Die enzymatischen Haarfärbemittel des Standes der Technik weisen ferner den Nachteil auf, daß relativ hohe Enzymmengen zur Erzielung üblicher Färbeergebnisse benötigt werden.

Die vorliegende Erfindung geht daher von der Aufgabe aus, enzymatische Färbemittel für keratinische Fasern zur Verfügung zu stellen, die - auch bereits bei der Verwendung minimaler Enzym- und/oder Farbstoffmengen - die an Färbemittel gestellten Anforderungen in einem hohen Maße erfüllen. Es ist ferner eine Aufgabe der vorliegenden Erfindung Färbemittel zur Verfügung zu stellen, die schonend zu Haut und Haar sind.

Ein erster Gegenstand der vorliegenden Erfindung sind daher Mittel zum Färben keratinischer Fasern, die in einem kosmetisch akzeptablen Träger
(A) mindestens ein Farbstoffvorprodukt,
(B) mindestens ein Enzym, das die Oxidation der Farbstoffvorprodukte katalysieren kann, ausgewählt aus den Laccasen (EC-No. 1.10.3.2), den Tyrosinasen (EC-No. 1.10.3.1), den Ascorbat-Oxidase (EC-No. 1.10.3.3), den Bilirubin-Oxidasen (EC-No. 1.3.35) sowie den Phenoloxidasen von Typ Acremonia, Stachybotrys oder Pleurotus.
(C) mindestens ein Zuckertensid, ausgewählt aus der Gruppe, die gebildet wird von
   - Alkyl- und Alkenyloligoglykosiden und
   - Fettsäure-N-alkyl-polyhydroxyalkylamiden und
(D) mindestens ein Reduktionsmittel.
enthalten.

Unter keratinischen Fasern sind erfindungsgemäß Pelze, Wolle, Federn und insbesondere menschliche Haare zu verstehen.

### Farbstoffvorprodukte

Hinsichtlich der in den erfindungsgemäßen Färbemitteln eingesetzten Farbstoffvorprodukte (A) unterliegt die vorliegende Erfindung keinerlei Einschränkungen. Die erfindungsgemäßen Färbemittel können als Farbstoffvorprodukte
- Oxidationsfarbstoffvorprodukte vom Entwickler- und/oder Kuppler-Typ, und
- Vorstufen naturanaloger Farbstoffe, wie Indol- und Indolin-Derivate,
sowie Mischungen von Vertretern dieser Gruppen enthalten.

In einer ersten bevorzugten Ausführungsform enthält das Färbemittel mindestens eine Entwicklerkomponente. Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterocyclische Hydrazone, 4-Aminopyrazolderivate sowie 2,4,5,6-Tetraaminopyrimidin und dessen Derivate eingesetzt.

Es kann erfindungsgemäß bevorzugt sein, als Entwicklerkomponente ein p-Phenylendiaminderivat oder eines seiner physiologisch verträglichen Salze einzusetzen. Besonders bevorzugt sind p-Phenylendiaminderivate der Formel (E1) 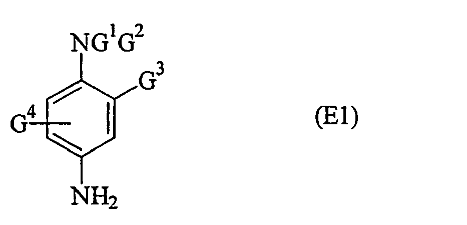 wobei
- G¹ steht für ein Wasserstoffatom, ein C₁- bis C₄-Alkylradikal, ein C₁- bis C₄-Monohydroxyalkylradikal, ein C₂- bis C₄-Polyhydroxyalkylradikal, ein (C₁- bis C₄)-Alkoxy-(C₁- bis C₄)-alkylradikal, ein 4'-Aminophenylradikal oder ein C₁- bis C₄-Alkylradikal, das mit einer stickstoffhaltigen Gruppe, einem Phenyl- oder einem 4'-Aminophenylrest substituiert ist;
- G² steht für ein Wasserstoffatom, ein C₁- bis C₄-Alkylradikal, ein C₁- bis C₄-Monohydroxyalkylradikal, ein C₂- bis C₄-Polyhydroxyallcylradikal, ein (C₁- bis C₄)-Alkoxy-(C₁- bis C₄)-alkylradikal oder ein C₁- bis C₄-Alkylradikal, das mit einer stickstoffhaltigen Gruppe substituiert ist;
- G³ steht für ein Wasserstoffatom, ein Halogenatom, wie ein Chlor-, Brom, Jod- oder Fluoratom, ein C₁- bis C₄-Alkylradikal, ein C₁- bis C₄-Monohydroxyalkylradikal, ein C₁- bis C₄-Hydroxyalkoxyradikal, ein C₁- bis C₄-Acetylaminoalkoxyradikal, ein C₁- bis C₄- Mesylaminoalkoxyradikal oder ein C₁- bis C₄-Carbamoylaminoalkoxyradikal;
- G⁴ steht für ein Wasserstoffatom, ein Halogenatom oder ein C₁- bis C₄-Alkylradikal oder
- wenn G³ und G⁴ in ortho-Stellung zueinander stehen, können sie gemeinsam eine verbrückende α,ω-Alkylendioxogruppe, wie beispielsweise einen Ethylendioxygruppe bilden.

Beispiele für die als Substituenten in den erfindungsgemäßen Verbindungen genannten, C₁- bis C₄-Alkylradikale sind die Gruppen Methyl, Ethyl, Propyl, Isopropyl und Butyl. Ethyl und Methyl sind bevorzugte Alkylradikale. Erfindungsgemäß bevorzugte C₁- bis C₄-Alkoxyradikale sind beispielsweise eine Methoxy- oder eine Ethoxygruppe. Weiterhin können als bevorzugte Beispiele für eine C₁- bis C₄-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Beispiele für Halogenatome sind erfindungsgemäß F-, Cl- oder Br-Atome, Cl-Atome sind ganz besonders bevorzugt. Die weiteren verwendeten Begriffe leiten sich erfindungsgemäß von den hier gegebenen Definitionen ab. Beispiele für stickstoffhaltige Gruppen der Formel (E1) sind insbesondere die Aminogruppen, C₁- bis C₄-Monoalkylaminogruppen, C₁- bis C₄-Dialkylaminogruppen, C₁- bis C₄-Trialkylammoniumgruppen, C₁- bis C₄-Monohydroxy-alkylaminogruppen, Imidazolinium und Ammonium.

Besonders bevorzugte p-Phenylendiamine der Formel (E1) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-bis-(β-Hydroxyethyl)-p-phenylendiamin, 4-N,N-bis-(β-Hydroxyethyl)amino-2-methylanilin, 4-N,N-bis-(β-Hydroxyethyl)amino-2-chloranilin, 2-(β-Hydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenylp-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin und 5,8-Diaminobenzo-1,4-dioxan sowie ihren physiologisch verträglichen Salzen.

Erfindungsgemäß ganz besonders bevorzugte p-Phenylendiaminderivate der Formel (E1) sind p-Phenylendiamin, p-Toluylendiamin, 2-(β-Hydroxyethyl)-p-phenylendiamin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Färbezusammensetzungen gemäß der Erfindung verwendet werden können, kann man insbesondere die Verbindungen nennen, die der folgenden Formel (E2) entsprechen, sowie ihre physiologisch verträglichen Salze: 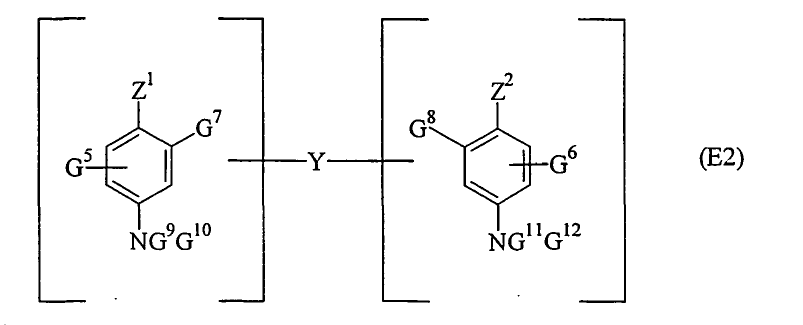 wobei:
- Z¹ und Z² stehen unabhängig voneinander für ein Hydroxyl- oder NH₂-Radikal, das gegebenenfalls durch ein C₁- bis C₄-Alkylradikal, durch ein C₁- bis C₄-Hydroxyalkylradikal und/oder durch eine Verbrückung Y substituiert ist oder das gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch ein oder mehrere Hydroxyl- oder C₁- bis C₈-Alkoxyradikale substituiert sein kann, oder eine direkte Bindung,
- G⁵ und G⁶ stehen unabhängig voneinander für ein Wasserstoff- oder Halogenatom, ein C₁- bis C₄-Alkylradikal, ein C₁- bis C₄-Monohydroxyalkylradikal, ein C₂- bis C₄-Polyhydroxyalkylradikal, ein C₁- bis C₄-Aminoalkylradikal oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder ein C₁- bis C₄-Alkylradikal, mit den Maßgaben, daß
- die Verbindungen der Formel (E2) nur eine Verbrückung Y pro Molekül enthalten und
- die Verbindungen der Formel (E2) mindestens eine Aminogruppe enthalten, die mindestens ein Wasserstoffatom trägt.

Die in Formel (E2) verwendeten Substituenten sind erfindungsgemäß analog zu den obigen Ausführungen definiert.

Bevorzugte zweikernige Entwicklerkomponenten der Formel (E2) sind insbesondere: N,N'-bis-(β-Hydroxyethyl)-N,N'-bis-(4'-aminophenyl)-1,3-diamino-propan-2-ol, N,N'- bis-(β-Hydroxyethyl)-N,N'-bis-(4'-aminophenyl)-ethylendiamin, N,N'-bis-(4-Aminophenyl)-tetramethylendiamin, N,N'-bis-(β-Hydroxyethyl)-N,N'-bis-(4-aminophenyl)-tetramethylendiamin, N,N'-bis-(4-Methyl-aminophenyl)-tetramethylendiamin, N,N'-bis-(Ethyl)-N,N'-bis-(4'-amino-3'-methylphenyl)-ethylendiamin, Bis-(2-hydroxy-5-aminophenyl)-methan, 1,4-Bis-(4'-aminophenyl)-diaza-cycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan und ihre physiologisch verträglichen Salze.

Ganz besonders bevorzugte zweikernige Entwicklerkomponenten der Formel (E2) sind N,N'-bis-(β-Hydroxyethyl)-N,N'-bis-(4'-aminophenyl)-1,3-diamino-propan-2-ol, Bis-(2-hydroxy-5-aminophenyl)-methan, N,N'-Bis-(4'-aminophenyl)-1,4-diazacycloheptan und 1,10-Bis-(2',5'-diaminophenyl)-1,4,7,10-tetraoxadecan oder eines ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (E3) 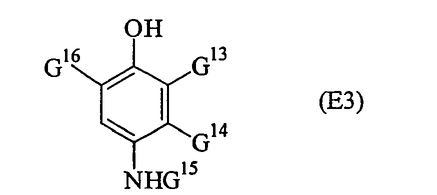 wobei:
- G¹³ steht für ein Wasserstoffatom, ein Halogenatom, ein C₁- bis C₄-Alkylradikal, ein C₁- bis C₄-Monohydroxyalkylradikal, ein (C₁- bis C₄)-Alkoxy-(C₁- bis C₄)-alkylradikal, ein C₁- bis C₄-Aminoalk-ylradikal, ein Hydroxy-(C₁- bis C₄)-alkylaminoradikal, ein C₁- bis C₄-Hydroxyalkoxyradikal, ein C₁- bis C₄-Hydroxyalkyl-(C₁-bis C₄)-aminoalkylradikal oder ein (Di-C₁- bis C₄-Alkylamino)-(C₁- bis C₄)-alkylradikal, und
- G¹⁴ steht für ein Wasserstoff- oder Halogenatom, ein C₁- bis C₄-Alkylradikal, ein C₁ - bis C₄-Monohydroxyalkylradikal, ein C₂- bis C₄-Polyhydroxyalkylradikal, ein (C₁- bis C₄)-Alkoxy-(C₁- bis C₄)-alkylradikal, ein C₁- bis C₄-Aminoalkylradikal oder ein C₁- bis C₄-Cyanoalkylradikal,
- G¹⁵ steht für Wasserstoff, ein C₁- bis C₄-Allcylradikal, ein C₁- bis C₄-Mono-hydroxyalkylradikal, ein C₂- bis C₄-Polyhydroxyalkylradikal, ein Phenylradikal oder ein Benzylradikal, und
- G¹⁶ steht für Wasserstoff oder ein Halogenatom.

Die in Formel (E3) verwendeten Substituenten sind erfindungsgemäß analog zu den obigen Ausführungen definiert.
Bevorzugte p-Aminophenole der Formel (E3) sind insbesondere p-Aminophenol, N-Methyl-p-Aminophenol, 4-Amino-3-methyl-phenol, 4-Amino-3-fluorphenol, 2-Hydroxy-methylamino-4-amino-phenol, 4-Amino-3-hydroxymethylphenol, 4-Amino-2-(2-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-fluorophenol, 4-Amino-2-chlorphenol, 2,6-Dichlor-4-aminophenol, 4-Amino-2-((diethylamino)methyl)phenol sowie ihre physiologisch verträglichen Salze.

Ganz besonders bevorzugte Verbindungen der Formel (E3) sind p-Aminophenol, 4-Amino-3-methylphenol, 4-Amino-2-aminomethylphenol und 4-Amino-2-((diethyl-amino)methyl)phenol.

Ferner kann die Entwicklerkomponente ausgewählt sein aus o-Aminophenol und seinen Derivaten, wie beispielsweise 2-Amino-4-methylphenol oder 2-Amino-4-chlorphenol.

Weiterhin kann die Entwicklerkomponente ausgewählt sein aus heterocyclischen Entwicklerkomponenten, wie beispielsweise den Pyridin-, Pyrimidin-, Pyrazol-, Pyrazol-Pyrimidin-Derivaten und ihren physiologisch verträglichen Salzen.

Bevorzugte Pyridin-Derivate sind insbesondere die Verbindungen, die in den Patenten GB 1 026 978 und GB 1 153 196 beschrieben werden, wie 2,5-Diamino-pyridin, 2-(4-Methoxyphenyl)amino-3-amino-pyridin, 2,3-Diamino-6-methoxy-pyridin, 2-(β-Methoxyethyl)amino-3-amino-6-methoxy-pyridin und 3,4-Diamino-pyridin.

Bevorzugte Pyrimidin-Derivate sind insbesondere die Verbindungen, die im deutschen Patent DE 2 359 399, der japanischen Offenlegungsschrift JP 02019576 A2 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere die Verbindungen, die in den Patenten DE 3 843 892, DE 4 133 957 und Patentanmeldungen WO 94/08969, WO 94/08970, EP-740931 und DE 195 43 988 beschrieben werden, wie 4,5-Diamino-1-methylpyrazol, 4,5-Diamino-1-(β-hydroxyethyl)-pyrazol, 3,4-Diaminopyrazol, 4,5-Diamino-1-(4'-chloro-benzyl)-pyrazol, 4,5-Diamino-1,3-dimethylpyrazol, 4,5-Diamino-3-methyl-l-phenyl-pyrazol, 4,5-Diamino-l-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-l-methylpyrazol, 4,5-Diamino-1-tert.-butyl-3-methylpyrazol, 4,5-Diamino-1-(β-hydroxyethyl)-3-methylpyrazol, 4,5-Diamino-l-ethyl-3-methylpyrazöl, 4,5-Diamino-l-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4, 5-Diamino-3-hydroxymethyl-1-isopropylpyrazol, 4,5-Diamino-3-methyl-1-isopropylpyrazol, 4-Amino-5-(2'-aminoethyl)amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4(β-hydroxyethyl)amino-1-methylpyrazol.

Bevorzugte Pyrazol-Pyrimidin-Derivate sind insbesondere die Derivate des Pyrazol-[1,5-a]-pyrimidin der folgenden Formel (E4) und dessen tautomeren Formen, sofern ein tautomerisches Gleichgewicht besteht: 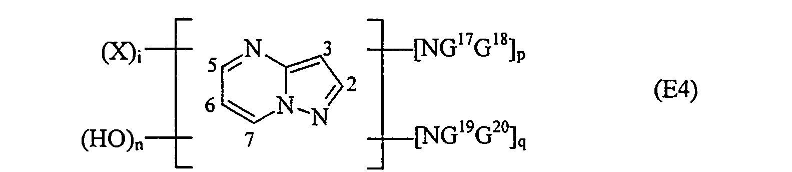 wobei:
- G¹⁷, G¹⁸, G¹⁹ und G²⁰ unabhängig voneinander stehen für ein Wasserstoffatom, ein C₁- bis C₄-Alkylradikal, ein Aryl-Radikal, ein C₁- bis C₄-Hydroxyalkylradikal, ein C₂- bis C₄-Polyhydroxyalkylradikal ein (C₁- bis C₄)-Akoxy-(C₁- bis C₄)-alkylradikal, ein C₁- bis C₄-Aminoalkylradikal, das gegebenenfalls durch ein Acetyl-Ureid- oder Sulfonyl-Radikal geschütz sein kann, ein (C₁- bis C₄)-Alkylamino-(C₁- bis C₄)-alkylradikal, ein Di-[(C₁- bis C₄)-alkyl]-(C₁- bis C₄)-aminoalkylradikal, wobei die Dialkyl-Radikale gegebenenfalls einen Kohlenstoffzyklus oder einen Heterozyklus mit 5 oder 6 Kettenglieden bilden, ein C₁- bis C₄-Hydroxyalkyl- oder ein Di-(C₁- bis C₄)-[Hydroyalkyl]-(C₁- bis C₄)-amino-alkylradikal,
- die X-Radikale stehen unabhängig voneinander für ein Wasserstoffatom, ein C₁- bis C₄-Alkylradikal, ein Aryl-Radikal, ein (C₁- bis C₄-Hydroxyalkyradikal, ein (C₂- bis C₄)-Polyhydroxyalkylradikal, ein [(C₁- bis C₄Aminoalkylradikal, ein (C₁- bis C₄)-Alkylamino- (C₁- bis C₄-aminoalkylradikal, ein Di-[(C₁- bis C₄)alkyl]-(C₁- bis C₄-aminoalkylradikal, wobei die Dialkyl-Radikale gegebenfalls einen Kohlenstoffzyklus oder einen Heterozyklus mit 5 oder 6 Kettengliedern bilden, ein (C₁- bis C₄-Hydroxyalkyl- oder ein Di-(C₁- bis C₄-hydroxyalkyl)-aminoalkylradikal, ein Aminoradikal, ein C₁- bis C₄-Alkyl- oder Di-(C₁- bis C₄-hydroxyalkyl)-aminoradikal, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,
mit der Maßgabe, daß
- die Summe aus p + q ungleich 0 ist,
- wenn p + q gleich 2 ist, n den Wert 0 hat, und die Gruppen NG¹⁷G¹⁸ und NG¹⁹G²⁰ belegen die Positionen (2,3); (5,6); (6,7); (3,5) oder (3,7);
- wenn p + q gleich 1 ist, n den Wert 1 hat, und die Gruppen NG¹⁷G¹⁸ (oder NG¹⁹G²⁰) und die Gruppe OH belegen die Positionen (2,3); (5,6); (6,7); (3,5) oder (3,7);

Die in Formel (E4) verwendeten Substituenten sind erfindungsgemäß analog zu den obigen Ausführungen definiert.

Wenn das Pyrazol-[1,5-a]-pyrimidin der obenstehenden Formel (E4) eine Hydroxygruppe an einer der Positionen 2, 5 oder 7 des Ringsystems enthält, besteht ein tautomers Gleichgewicht, das zum Beispiel im folgenden Schema dargestellt wird:

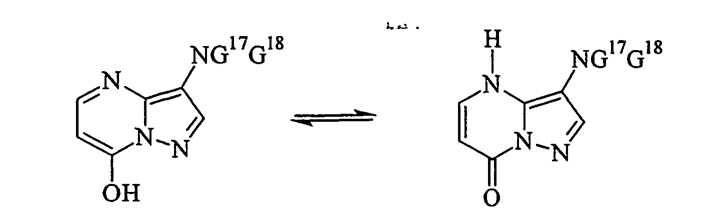

Unter den Pyrazol-[1,5-a]-pyrimidinen der obenstehenden Formel (E4) kann man insbesondere nennen:
- Pyrazol-[1,5-a]-pyrimidin-3,7-diamin;
- 2,5-Dimethyl pyrazol-[1,5-a]-pyrimidin-3,7-diamin;
- Pyrazol-(1,5-a]-pyrimidin-3,5-diamin;
- 2,7-Dimethyl pyrazol-[1,5-a]-pyrimidin-3,5-diamin;
- 3-Amino pyrazol-[1,5-a]-pyrimidin-7-ol;
- 3-Amino pyrazol-[1,5-a]-pyrimidin-5-ol;
- 2-(3-Amino pyrazol-[1,5-a]-pyrimidin-7-ylamino)-ethanol;
- 2-(7-Amino pyrazol-[1,5-a]-pyrimidin-3-ylamino)-ethanol;
- 2-[(3-Amino pyrazol-[1,5-a]-pyrimidin-7-yl)-(2-hydroxy-ethyl)-amino]-ethanol;
- 2-[(7-Amino pyrazol-[1,5-a]-pyrimidin-3-yl)-(2-hydroxy-ethyl)-amino]-ethanol;
- 5,6-Dimethyl pyrazol-[1,5-a]-pyrimidin-3,7-diamin;
- 2,6-Dimethyl pyrazol-[1,5-a]-pyrimidin-3,7-diamin;
- 2,5, N7, N7-Tetramethyl pyrazol-[1,5-a]-pyrimidin-3,7-diamin;
sowie ihre physiologisch verträglichen Salze und ihre tautomeren Formen, wenn ein tautomerisches Gleichgewicht vorhanden ist.

Die Pyrazol-[1,5-a]-pyrimidine der obenstehenden Formel (E4) können wie in der Literatur beschrieben durch Zyklisierung ausgehend von einem Aminopyrazol oder von Hydrazin hergestellt werden.

Ferner können die erfindungsgemäßen Mittel kationische Farbstoffvorprodukte vom Kuppler- und/oder Entwicklertyp enthalten, wie sie beispielsweise in den Offenlegungsschriften WO-A1-99/03 819, WO-A2-99/03 834, WO-A1-99/03 836, WO-A1-99/48 856, WO-A1-99/48 874, WO-A1-99/48 875, WO-A2-00/42 971, WO-A1-00/42 979, WO-A1-00/42 980, WO-A1-00/43 356, WO-A1-00/43 367, WO-A1-00/43 368, WO-A1-00/43 386, WO-A1-00/43 388, WO-A1-00/43 389, WO-Al-00/43 396, EP-A1-0 984 006, EP-A1-0 984 007 und EP-A1-0 989 128 beschrieben werden.

Besonders bevorzugte kationische Farbstoffvorprodukte sind
[2-(2',5'-Diamino-phenoxy)-ethyl]-diethyl-methyl-ammonium-chlorid,
[2-(4'-Amino-phenylamino)-propyl]-trimethyl-ammonium-chlorid,
[4-(4'-Amino-phenylamino)-pentyl]-diethyl-(2-hydroxyethyl)-ammonium-chlorid,
1-{[5'-Amino-2'-(2"-hydroxyethylamino)-phenylcarbamoyl]-methyl}-1,4-dimethylpiperazin-1-ium-chlorid,
1,4-Bis-1-{3-[3'-(2",5"-diamino-phenoxy)-propyl]-3H-imidazol-1-ium}-butan-dichlorid,
1,3-Bis-[3'-(2",5"-diamino-phenoxy)-propyl]-3H-imidazol-1-ium-chlorid
N,N'-Bis-[3-N-methyl-4-N-(4'-amino-anilin)-ethyl]-1,1,4,4-tetramethyl-diammonium-1,3-propan-dibromid,
1,3-Bis-1-{3-{3'-[(4"-amino-3"-methyl-anilin)-N-propyl] -3H-imidazol-1-ium} -propan-dichlorid
1,3-Bis-1-{3-{3'-[(4"-amino-anilin)-N-propyl]}-3H-imidazol-1-ium} -propan-dichlorid
1,3-Bis-1-{3-{3'-[(4"-amino-2"-methyl-anilin)-N-propyl] }-3H-imidazol-1-ium}-propan-dichlorid
3-[3-(4'-Amino-phenylamino)-propyl]-1-methyl-3H-imidazol-1-ium-chlorid
[3-(2',5'-Diamino-phenoxy)-propyl]-3-methyl-3H-imidazol-1-ium-chlorid
3-[3-(4'-Amino-3'-methyl-phenylamino)-propyl]-1-methyl-3H-imidazol-1-ium-chlorid
3-[3-(4'-Amino-2'-methyl-phenylamino)-propyl]-1-methyl-3H-imidazol-1-ium-chlorid
1-[2-(4'-Amino-2'-methoxy-phenylamino)- ethyl]-3-methyl-3H-imidazol-1-ium-chlorid
3-[3-(4'-Amino-2'-fluoro-phenylamino)-propyl]-1-methyl-3H-imidazol-1-ium-chlorid
3-[3-(4'-Amino-2'-cyano-phenylamino)-propyl]-1-methyl-3 H-imidazol-1-ium-chlorid
3-[2-(2',5'-Diamino-phenyl)-ethyl]-1-methyl-3H-imidazol-1-ium-chlorid
1-{2-[(4'-Amino-phenyl)-ethyl-amino]-ethyl}-3-methyl-3H-imidazol-1-ium-chlorid
N,N-Bis-[2-(3'-methyl-3H-imidazol-1-ium)-ethyl]-4-amino-anilin-chlorid
3-[2-(4'-Amino-phenylamino)-butyl]-1-methyl-3H-imidazol-1-ium-chlorid
[2-(2',4'-Diamino-phenoxy)-ethyl]-diethyl-methyl-ammonium-chlorid
1-[3-(2',4'-Diamino-phenoxy)-propyl]-3-methyl-3H-imidazol-1-ium-chlorid
1-[(3'-Hydroxy-4'-methyl-phenylcarbamoyl)-methyl]-3-methyl-3H-imidazol-1-ium-chlorid
1,4-Bis-1-{3-[3-(2',4'-diamino-phenoxy)-propyl]-3H-imidazol-1-ium}-butan-dichlorid
3-[(3'-Hydroxy-4'-methansulfonylamino-phenylcarbamoyl)-methyl]-1-methyl-3H-imidazol-1-ium-chlorid
3-[(3',5'-Dichloro-2'-hydroxy-4'-methyl-phenylcarbamoyl)-methyl]-1-methyl-3 H-imidazol-1-ium-chlorid
1-[(3',5'-Dichloro-2'-hydroxy-4'-methyl-phenylcarbamoyl)-methyl]-1,4-dimethylpiperazin-1-ium-chlorid
3-[(4'-Acetylamino-2'-hydroxy-phenylcarbamoyl)-methyl]-1-methyl-3H-imidazol-1-umchlorid
4-{3-[(3'-Hydroxy-naphthalen-2'-carbonyl)-amino]-propyl}-4-methyl-morpholin-4-iumiodid
3-[(1'-Hydroxy-naphthalen-2'-ylcarbamoyl)-methyl]-1-methyl-3H-imidazol-1-ium-chlorid
3-[(5'-Acetylamino-1'-hydroxy-naphthalen-2'-ylcarbamoyl)-methyl]-1-methyl-3H-imidazol-1-ium-chlorid
3-[(1'-Hydroxy-5'-methanesulfonylamino-naphthalen-2'-ylcarbamoyl)-methyl]-1-methyl-3H-imidazol-1-ium-chlorid
[3-(4'-Amino-2',5'-dimethyl-2H-pyrazol-3'-ylamino)-propyl]-(2-hydroxyethyl)-dimethylammonium-chlorid
1,3-Bis-[(2'-hydroxy-4'-methyl-phenylcarbamoyl)-methyl]-3H-imidazol-1-ium-chlorid
1-[2-(6'-Amino-benzo[1,3]dioxol-5'-ylamino)-ethyl]-3-methyl-3H-imidazol-1-iumchlorid
3-[2-(6'-Amino-benzo[1,3]dioxol-5'-ylamino)-ethyl]-1-(4-{3-[2-(6"-aminobenzo[1,3]dioxol-5"-ylamino)-ethyl]-3H-imidazol-1-ium}-butyl)-3H-imidazol-1-iumdichlorid
3-[3 -(3'-Amino-5'-methyl-pyrazolo[1,5-a]pyrimidin-7'-ylamino)-propyl]-1-(2-hydroxyethyl)-3H- imidazol-1-ium-chlorid
1,3-Bis-1-{3-{3-[(2'-amino-anilin)-N-propyl]}-3H-imidazol-1-ium}-propan-dibromid
N,N'-Bis-[3-N-(2'-amino-anilin)-N-propyl]-1,1,3,3-tetramethyl-diammonium-1,3-propandibromid
3-[3-(2'-Aminophenylamino)-propyl]-1-methyl-3H-imidazol-1-ium-chlorid
[2-(2'-Aminophenylamino)-ethyl]-trimethylammonium-chlorid und
3-(4'-Hydroxy-1'-methyl-1H-indol-5'-ylmethyl)-1-methyl-pyridinium-methosulfat.

Weiterhin sind erfindungsgemäß Färbemittel bevorzugt, die mindestens eine Kupplerkomponente enthalten. Als Kupplerkomponenten werden in der Regel m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methylpyrazolon-5, 2,4-Dichlor-3-aminophenol, 1,3-Bis-(2,4-diaminophenoxy)-propan, 2-Chlorresorcin, 4-Chlor-resorcin, 2-Chlor-6-methyl-3-aminophenol, 2-Amino-3-hydroxypyridin, 2-Methylresorcin, 5-Methylresorcin und 2-Methyl-4-chlor-5-aminophenol.

Erfindungsgemäß bevorzugte Kupplerkomponenten sind:
- m-Aminophenol und dessen Derivate wie beispielsweise 5-Amino-2-methylphenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-(Ethylamino)-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2,4-diaminophenoxy)-propan, 1-Methoxy-2-amino-4-(2'-hydroxyethylamino)benzol, 1,3-Bis-(2,4-diaminophenyl)-propan, 2,6-Bis-(2-hydroxyethyl-amino)-1-methylbenzol und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2;6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol.

Besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Es ist nicht erforderlich, daß die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Sofern es sich bei den Farbstoffvorprodukten um Amino-Verbindungen handelt, lassen sich aus diesen in üblicher Weise die bekannten Säureadditionssalze herstellen. Alle Aussagen dieser Schrift und demgemäß der beanspruchte Schutzbereich beziehen sich daher sowohl auf die in freier Form vorliegenden Verbindungen als auch auf deren wasserlösliche, physiologisch verträgliche Salze. Beispiele für solche Salze sind die Hydrochloride, die Hydrobromide, die Sulfate, die Phosphate, die Acetate, die Propionate, die Citrate und die Lactate.

Die Oxidationsfarbstoffvorprodukte sind in den erfindungsgemäßen Mitteln bevorzugt in Mengen von 0,01 bis 20 Gew.-%, vorzugsweise 0,5 bis 5 Gew.-%, jeweils bezogen auf das gesamte Mittel, enthalten.

Als Vorstufen naturanaloger Farbstoffe werden bevorzugt solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer zweiten bevorzugten Ausführungsform enthalten die Färbemittel mindestens ein Indol- und/oder Indolinderivat.

Besonders gut als Vorstufen naturanaloger Haarfarbstoffe geeignet sind Derivate des 5,6-Dihydroxyindolins der Formel (Ia), 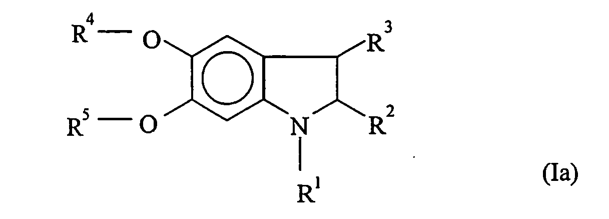 in der unabhängig voneinander
- R¹ steht für Wasserstoff, eine C₁-C₄-Alkylgruppe oder eine C₁-C₄-Hydroxy-alkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel (Ib), 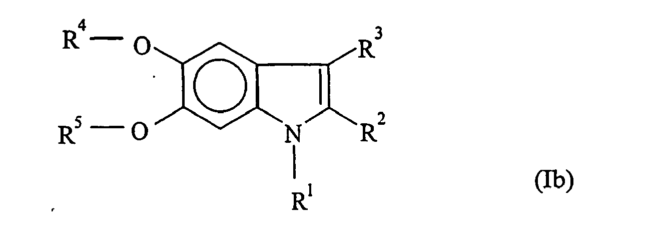 in der unabhängig voneinander
- R¹ steht für Wasserstoff, eine C₁-C₄-Alkylgruppe oder eine C₁-C₄-Hydroxyalkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
- sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate können in den im Rahmen des erfindungsgemäßen Verfahrens eingesetzten Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden. Die Indol- oder Indolin-Derivate sind in diesen üblicherweise in Mengen von 0,05-10 Gew.-%, vorzugsweise 0,2-5 Gew.-% enthalten.

In einer weiteren Ausführungsform kann es erfindungsgemäß bevorzugt sein, das Indolin- oder Indolderivat in Haarfärbemitteln in Kombination mit mindestens einer Aminosäure oder einem Oligopeptid einzusetzen. Die Aminosäure ist vorteilhafterweise eine α-Aminosäure; ganz besonders bevorzugte α-Aminosäuren sind Arginin, Ornithin, Lysin, Serin und Histidin, insbesondere Arginin.

Neben den Farbstoffvorprodukten können die erfindungsgemäßen Mittel zur Erzielung der gewünschten färberischen Effekte weiterhin mindestens einen direktziehenden Farbstoff enthalten. Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 12, Disperse Blue 3, HC Violet 1, Disperse Violet 1, Disperse Violet 4, Acid Violet 43, Disperse Black 9 und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Hydroxyethyl-2-nitro-toluidin, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Ferner können die erfindungsgemäßen Mittel einen kationischen direktziehenden Farbstoff enthalten. Besonders bevorzugt sind dabei
(i) kationische Triphenylmethanfarbstoffe, wie beispielsweise Basic Blue 7, Basic Blue 26, Basic Violet 2 und Basic Violet 14,
(ii) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(iii) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (iii) sind insbesondere die folgenden Verbindungen:

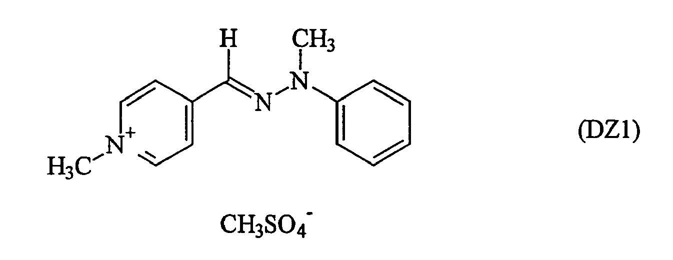

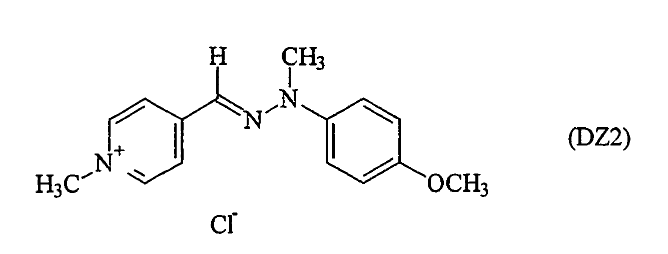

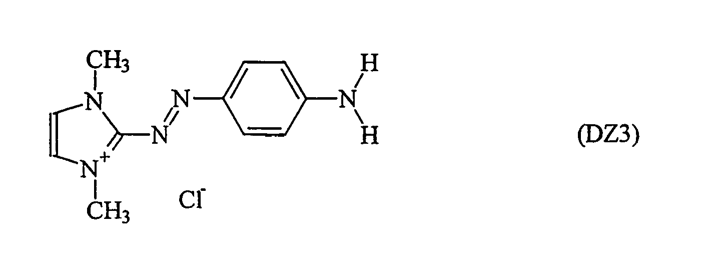

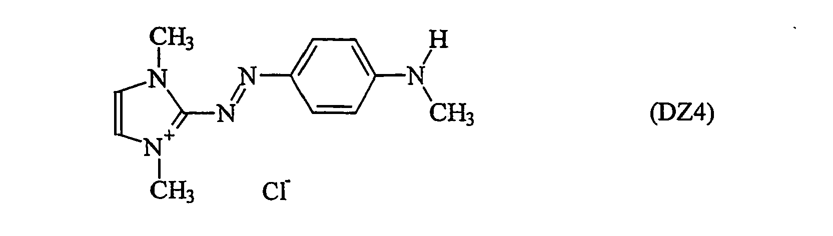

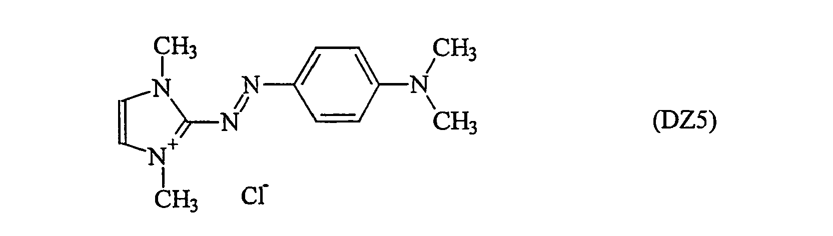

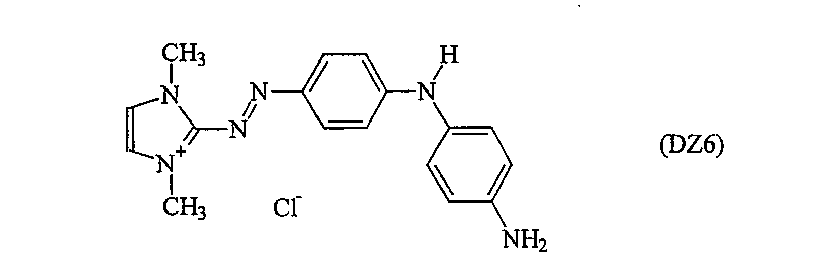

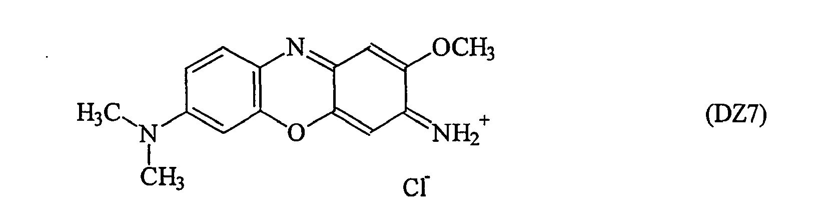

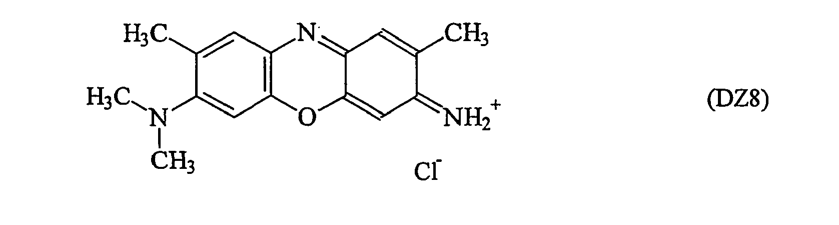

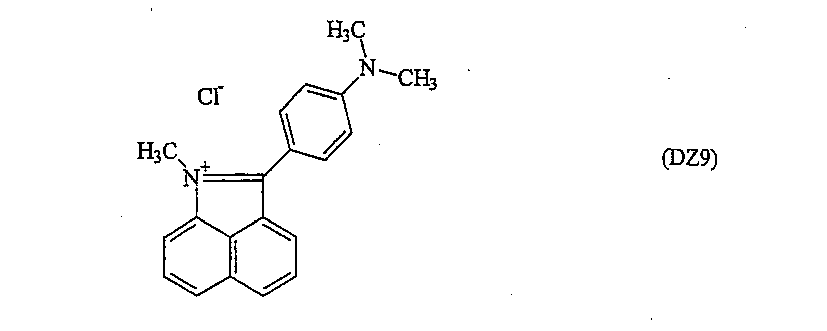

Die Verbindungen der Formeln (DZ1), (DZ3) und (DZ5) sind ganz besonders bevorzugte kationische direktziehende Farbstoffe der Gruppe (üi).

Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

### Enzyme

Ferner enthalten die erfindungsgemäßen Mittel als zweite zwingende Komponente mindestens ein Enzym, das die Oxidation der Farbstoffvorprodukte katalysieren kann.

Die einzusetzenden Enzyme, die mithilfe von Luftsauerstoff die Farbstoffvorprodukte direkt oxidieren, sind die Laccasen (EC-Nr. 1.10.3.2), die Tyrosinasen (EC-Nr. 1.10.3.1), Ascorbat-Oxidase (EC-Nr. 1.10.3.3), die Bilirubin-Oxidasen (BC-Nr. 1.3.3.5) sowie Phenoloxidasen des Typs Acremonia, Stachybotrys oder Pleurotus. Die Laccasen sind erfindungsgemäß ganz besonders bevorzugte Enzyme.

Die Enzyme können in das Färbemittel selbst eingearbeitet werden; bevorzugt werden sie aber getrennt von den Farbstoffvorprodukten konfektioniert und erst unmittelbar vor der Anwendung mit dem eigentlichen Färbemittel vermischt. Sollten die Enzyme als 1-Komponentensystem in das Färbemittel eingearbeitet werden, ist es erfindungsgemäß bevorzugt, das System unter Luftausschluß zu konfektionieren.

Ferner hat sich gezeigt, daß es erfindungsgemäß bevorzugt sein kann, das Färbemittel frei von Treibmitteln, daß heißt nicht als Schaumformulierung zu konfektionieren.

Das Enzym wird bevorzugt in einer Menge von 0,0001 - 1 Gew.-%, bezogen auf die Proteinmenge des Enzyms und das gesamte Färbemittel, eingesetzt.

### Zuckertenside

Die erfindungsgemäßen Mittel enthalten als dritte zwingende Komponente (C) mindestens ein Zuckertensid.

Gemäß einer ersten Ausführungsform (C1) handelt es sich bei dem Zuckertensid um ein Alkyl- oder Alkenyloligoglykosid. Diese Zuckertenside stellen bekannte nichtionische Tenside gemäß Formel (II) dar,

R¹O-[G]ₚ (II)

in der R¹ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Stellvertretend für das umfangreiche Schrifttum sei hier auf die Übersichtsarbeit von Biermann et al. in Starch/Stärke 45, 281 (1993), B. Salka in Cosm.Toil. 108, 89 (1993) sowie J. Kahre et al. in SÖFW-Journal Heft 8, 598 (1995) verwiesen.

Die Alkyl- und Alkenyloligoglykoside können sich von Aldosen beziehungsweise Ketosen mit 5 oder 6 Kohlenstoffatomen, vorzugsweise von Glucose, ableiten. Die bevorzugten Alkyl- und/oder Alkenyloligoglykoside sind somit Alkyl- und/oder Alkenyloligoglucoside. Die Indexzahl p in der allgemeinen Formel (II) gibt den Oligomerisierungsgrad (DP), d. h. die Verteilung von Mono- und Oligoglykosiden an und steht für eine Zahl zwischen 1 und 10. Während p im einzelnen Molekül stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl ist. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt.

Der Alkyl- bzw. Alkenylrest R¹ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestem oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R¹ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.

Gemäß einer zweiten Ausführungsform (C2) der Erfindung handelt es sich bei dem Zuckertensid um ein Fettsäure-N-alkylpolyhydroxyalkylamid, ein nichtionisches Tensid der Formel (III), 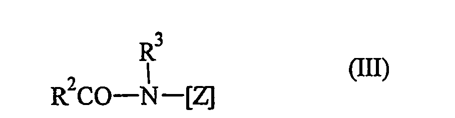 in der R²CO für einen aliphatischen Acylrest mit 6 bis 22 Kohlenstoffatomen, R³ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht.
Bei den Fettsäure-N-alkylpolyhydroxyalkylamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können. Hinsichtlich der Verfahren zu ihrer Herstellung sei auf die US-Patentschriften US 1,985,424, US 2,016,962 und US 2,703,798 sowie die Internationale Patentanmeldung WO 92/06984 verwiesen. Eine Übersicht zu diesem Thema von H. Kelkenberg findet sich in Tens. Surf. Det. 25, 8 (1988). Vorzugsweise leiten sich die Fettsäure-N-alkylpolyhydroxyalkylamide von reduzierenden Zuckern mit 5 oder 6 Kohlenstoffatomen, insbesondere von der Glucose, ab. Die bevorzugten Fettsäure-N-alkylpolyhydroxyalkylamide stellen daher Fettsäure-N-alkylglucamide dar, wie sie durch die Formel (IV) wiedergegeben werden: 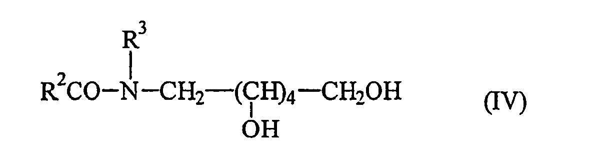 Vorzugsweise werden als Fettsäure-N-alkylpolyhydroxyalkylamide Glucamide der Formel (IV) eingesetzt, in der R³ für Wasserstoff oder eine Alkylgruppe steht und R²CO für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. derer technischer Mischungen steht. Besonders bevorzugt sind Fettsäure-N-alkylglucamide der Formel (IV), die durch reduktive Aminierung von Glucose mit Methylamin und anschließende Acylierung mit Laurinsäure oder C_{12/14}-Kokosfettsäure bzw. einem entsprechenden Derivat erhalten werden. Weiterhin können sich die Polyhydroxyalkylamide auch von Maltose und Palatinose ableiten.

Das Zuckertensid ist in den erfindungsgemäß verwendeten Mitteln bevorzugt in Mengen von 0,1 - 20 Gew.-%, bezogen auf das gesamte Mittel, enthalten. Mengen von 0,5 - 5 Gew.-% sind besonders bevorzugt.

### Reduktionsmittel

Weiterhin enthalten die erfindungsgemäßen Mittel ein Reduktionsmittel. Beispiele für erfindungsgemäß bevorzugte Reduktionsmittel sind Natriumsulfit, Ascorbinsäure, Thioglykolsäure und deren Derivate, Natriumthionit, Alkalimetallcitratsalze und N-Acetyl-L-Cystein Ganz besonders bevorzugte Reduktionsmittel sind Alkalimetallcitratsalze, insbesondere Natriumcitrat, und N-Acetyl-L-Cystein. N-Acetyl-L-Cystein ist ein ganz besonders bevorzugtes Alkalisierungsmittel.

### Fettsäurepartialglycerid

Als weitere Komponente können die erfindungsgemäßen Mittel Fettsäurepartialglyceride enthalten. Diese Fettsäurepartialglyceride sind Monoglyceride, Diglyceride und deren technische Gemische. Bei der Verwendung technischer Produkte können herstellungsbedingt noch geringe Mengen Triglyceride enthalten sein. Die Partialglyceride folgen vorzugsweise der Formel (V), 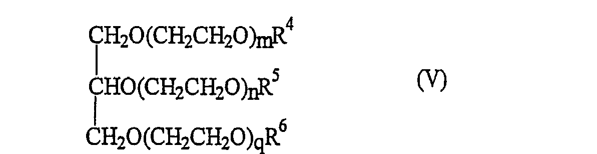 in der R⁴, R⁵ und R⁶ unabhängig voneinander für Wasserstoff oder für einen linearen oder verzweigten, gesättigten und/oder ungesättigten Acylrest mit 6 bis 22, vorzugsweise 12 bis 18, Kohlenstoffatomen stehen mit der Maßgabe, daß mindestens eine dieser Gruppen für einen Acylrest und mindestens eine dieser Gruppen für Wasserstoff steht. Die Summe (m+n+q) steht für 0 oder Zahlen von 1 bis 100, vorzugsweise für 0 oder 5 bis 25. Bevorzugt steht R⁴ für einen Acylrest und R⁵ und R⁶ für Wasserstoff und die Summe (m+n+q) ist 0. Typische Beispiele sind Mono- und/oder Diglyceride auf Basis von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden Ölsäuremonoglyceride eingesetzt.

Das Fettsäurepartialglycerid ist in den erfindungsgemäß verwendeten Mitteln bevorzugt in Mengen von 0,1 - 20 Gew.-%, insbesondere 0,5 - 2 Gew.-%, bezogen auf das gesamte Mittel enthalten.
Die erfindungsgemäße Lehre umfaßt auch Ausführungsformen, in denen die erfindungsgemäßen Mittel mehrere Zuckertenside und/oder mehrere Fettsäurepartialglyceride enthalten.

### Verdickungsmittel

Als weitere optionale Komponente können die erfindungsgemäßen Mittel ferner mindestens ein Verdickungsmittel enthalten. Bezüglich dieser Verdickungsmittel bestehen keine prinzipiellen Einschränkungen. Obwohl prinzipiell sowohl organische als auch rein anorganische Verdickungsmittel zum Einsatz kommen können, sind die organischen Verdickungsmittel erfindungsgemäß bevorzugt.

Gemäß einer ersten bevorzugten Ausführungsform handelt es sich bei dem Verdickungsmittel um ein anionisches, synthetisches Polymer. Bevorzugte anionische Gruppen sind die Carboxylat- und die Sulfonatgruppe.

Beispiele für anionische Monomere, aus denen die polymeren anionischen Verdickungsmittel bestehen können, sind Acrylsäure, Methacrylsäure, Crotonsäure, Itaconsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind Maleinsäureanhydrid sowie insbesondere 2-Acrylamido-2-methylpropansulfonsäure und Acrylsäure.

Bevorzugte anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichnen Carbopol^{®} im Handel erhältlich. Ebenfalls bevorzugt ist das Homopolymer der 2-Acrylamido-2-methylpropansulfonsäure, das beispielsweise unter der Bezeichnung Rheothik^{®}11-80 im Handel erhältlich ist.

Innerhalb dieser ersten Ausführungsform kann es weiterhin bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Itaconsäuremono- und -diester, Vinylpyrrolidon, Vinylether und Vinylester.

Bevorzugte anionische Copolymere sind beispielsweise Copolymere aus Acrylsäure, Methacrylsäure oder deren C₁- bis C₆-Alkylestern, wie sie unter der INCI-Deklaration Acrylates Copolymere vertrieben werden. Ein bevorzugtes Handelsprodukt ist beispielsweise Aculyn^{®} 33 der Firma Rohm & Haas. Weiterhin bevorzugt sind aber auch Copolymere aus Acrylsäure, Methacrylsäure oder deren C₁- bis C₆-Alkylestern und den Estern einer ethylenisch ungesättigten Säure und einem alkoxylierten Fettalkohol. Geeignete ethylenisch ungesättigte Säuren sind insbesondere Acrylsäure, Methacrylsäure und Itaconsäure; geeignete alkoxylierte Fettalkohole sind insbesondere Steareth-20 oder Ceteth-20. Derartige Copolymere werden von der Firma Rohm & Haas unter der Handelsbezeichnung Aculyn^{®} 22 sowie von der Firma National Starch unter den Handelsbezeichnungen Structure^{®} 2001 und Structure^{®} 3001 vertrieben.

Bevorzugte anionische Copolymere sind weiterhin Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vernetzungsagentien bevorzugt polyolefinisch ungesättigte Verbindungen wie Tetraallyloxythan, Allylsucrose, Allylpentaerythrit und Methylen-bisacrylamid zum Einsatz kommen. Ein solches Polymer ist in den Handelsprodukten Sepigel^{®}305 und Simulgel^{®} 600 der Firma SEPPIC enthalten. Die Verwendung dieser Compounds, die neben der Polymerkomponente eine Kohlenwasserstoffmischung (C₁₃-C₁₄-Isoparaffin beziehungsweise Isohexadecan) und einen nichtionogenen Emulgator (Laureth-7 beziehungsweise Polysorbate-80) enthalten, hat sich im Rahmen der erfindungsgemäßen Lehre als besonders vorteilhaft erwiesen.

Auch Polymere aus Maleinsäureanhydrid und Methylvinylether, insbesondere solche mit Vernetzungen, sind bevorzugte Verdickungsmittel. Ein mit 1,9-Decadien vernetztes Maleinsäure-Methylvinylether-Copolymer ist unter der Bezeichnungg Stabileze^{®} QM im Handel erhältlich.

Gemäß einer zweiten Ausführungsform handelt es sich bei dem Verdickungsmittel um einen kationisches synthetisches Polymer. Bevorzugte kationische Gruppen sind quartäre Ammoniumgruppen. Insbesondere solche Polymere, bei denen die quartäre Ammoniumgruppe über eine C₁₋₄-Kohlenwasserstoffgruppe an eine aus Acrylsäure, Methacrylsäure oder deren Derivaten aufgebaute Polymerhauptkette gebunden sind, haben sich als besonders geeignet erwiesen.

Homopolymere der allgemeinen Formel (VI), 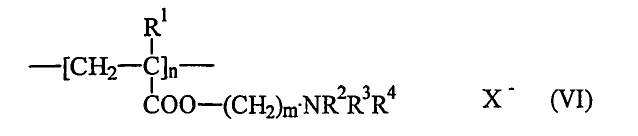 in der R¹ = -H oder -CH₃ ist, R², R³ und R⁴ unabhängig voneinander ausgewählt sind aus C₁₋₄-Alkyl-, -Alkenyl- oder -Hydroxyalkylgruppen, m = 1, 2, 3 oder 4, n eine natürliche Zahl und X⁻ ein physiologisch verträgliches organisches oder anorganisches Anion ist, sowie Copolymere, bestehend im wesentlichen aus den in Formel (I) aufgeführten Monomereinheiten sowie nichtionogenen Monomereinheiten, sind besonders bevorzugte kationische polymere Verdickungsmittel. Im Rahmen dieser Polymeren sind diejenigen erfindungsgemäß bevorzugt, für die mindestens eine der folgenden Bedingungen gilt:
- R¹ steht für eine Methylgruppe
- R², R³ und R⁴ stehen für Methylgruppen
- m hat den Wert 2,

Als physiologisch verträgliches Gegenionen X⁻ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloyloxyethyltrimethylammoniumchlorid) mit der INCI-Bezeichnung Polyquatemium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vemetzungsagens.

Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Salcare^{®} SC 95 (ca. 50 % Polymeranteil, weitere Komponente: Mineralöl (INCI-Bezeichnung: Mineral Oil) und Tridecyl-polyoxypropylen-polyoxyethylen-ether (INCI-Bezeichnung: PPG-1-Trideceth-6)) und Salcare^{®} SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylenether (INCI-Bezeichnung: PPG-1-Trideceth-6) im Handel erhältlich.

Weiterhin erfindungsgemäß geeignet sind nichtionische Verdickungsmittel vom Typ der polymeren Fettsäureamide, wie sie beispielsweise in der EP-A1-632083 oder des EP-A2-959066 offenbart werden.

Copolymere mit Monomereinheiten gemäß Formel (VI) enthalten als nichtionogene Monomereinheiten bevorzugt Acrylamid, Methacrylamid, Acrylsäure-C₁₋₄-Alkylester und Methacrylsäure-C₁₋₄-Alkylester. Unter diesen nichtionogenen Monomeren ist das Acrylamid besonders bevorzugt. Auch diese Copolymere können, wie im Falle der Homopolymeren oben beschrieben, vernetzt sein. Ein erfindungsgemäß bevorzugtes Copolymer ist das vernetzte Acrylamid- Methacryloyloxyethyltrimethylammoniumchlorid-Copolymer. Solche Copolymere, bei denen die Monomeren in einem Gewichtsverhältnis von etwa 20:80 vorliegen, sind im Handel als ca. 50 %ige nichtwäßrige Polymerdispersion unter der Bezeichnung Salcare^{®} SC 92 erhältlich.

In einer dritten bevorzugten Ausführungsform werden natürlich vorkommende Verdickungsmittel eingesetzt. Bevorzugte Verdickungsmittel dieser Ausführungsform sind beispielsweise nichtionischen Guargums. Erfindungsgemäß können sowohl modifizierte als auch unmodifizierte Guargums zum Einsatz kommen. Nichtmodifizierte Guargums werden beispielsweise unter der Handelsbezeichnung Jaguar^{®} C von der Firma Rhone Poulenc vertrieben. Erfindungsgemäß bevorzugte modifizierte Guargums enthalten C₁-bis C₆-Hydroxyalkylgruppen. Bevorzugt sind die Gruppen Hydroxymethyl, Hydroxyethyl, Hydroxypropyl und Hydroxybutyl. Derart modifizierte Guargums sind im Stand der Technik bekannt und können beispielsweise durch Reaktion der Guargums mit Alkylenoxiden hergestellt werden. Der Grad der Hydroxyalkylierung, der der Anzahl der verbrauchten Alkylenoxidmoleküle im Verhältnis zur Zahl der freien Hydroxygruppen der Guargums entspricht, liegt bevorzugt zwischen 0,4 und 1,2. Derart modifizierte Guar Gums sind unter den Handelsbezeichnungen Jaguar^{®} HP8, Jaguar^{®} HP60, Jaguar^{®} HP120, Jaguar^{®} DC 293 und Jaguar^{®} HP105 der Firma Rhone Poulenc im Handel erhältlich.

Weiterhin geeignete natürliche Verdickungsmittel sind ebenfalls bereits aus dem Stand der Technik bekannt. Es wird daher explizit auf das Werk von Robert L. Davidson mit dem Titel "Handbook of Water soluble gums and resins", erschienen bei Mc Graw Hill Book Company (1980) verwiesen.

Gemäß dieser Ausführungsform bevorzugt sind weiterhin Biosaccharidgums mikrobiellen Ursprungs, wie die Skleroglucangums oder Xanthangums, Gums aus pflanzlichen Exsudaten, wie beispielsweise Gummi arabicum, Ghatti-Gummi, Karaya-Gummi, Tragant-Gummi, Carrageen-Gummi, Agar-Agar, Johannisbrotkernmehl, Pektine, Alginate, Stärke-Fraktionen und Derivate wie Amylose, Amylopektin und Dextrine.

Aber auch nichtionische, vollsynthetische Polymere, wie beispielsweise Polyvinylalkohol oder Polyvinylpyrrolidon, sind als erfindungsgemäße Verdickungsmittel einsetzbar. Bevorzugte nichtionische, vollsynthetische Polymere werden beispielsweise von der Firma BASF unter dem Handelsnamen Luviskol^{®} vertrieben.

Erfindungsgemäß bevorzugt sind anionische und nichtionische verdickende Polymere.

In einer ganz besonders bevorzugten Ausführungsform der vorliegenden Erfindung werden als Verdickungsmittel Cellulose und/oder Cellulosederivate, wie beispielsweise Methylcellulose, Carboxyalkylcellulosen und Hydroxyalkylcellulosen eingesetzt.

Bevorzugte Hydroxyalkylcellulosen sind insbesondere die Hydroxyethylcellulosen, die unter den Bezeichnungen Cellosize^{®} der Firma Amerchol, Natrosol^{®} der Firma Hercules oder Culminal^{®} und Benecel^{®} der Firma Aqualon vertrieben werden. Ebenfalls erfindungsgemäß geeignet sind die Hydroxypropylcellulosen, wie sie beispielsweise unter der Bezeichnung Klucel^{®} von der Firma Hercules vertrieben werden. Geeignete Carboxyalkylcellulosen sind insbesondere die Carboxymethylcellulosen, wie sie unter den Bezeichnungen Blanose^{®} von der Firma Aqualon, Aquasorb^{®} und Ambergum^{®} von der Firma Hercules und Cellgon^{®} von der Firma Montello vertrieben werden.

### Pflegende Polymere

In einer weiteren bevorzugten Ausführungsform enthalten die erfindungsgemäßen Mittel weiterhin mindestens ein pflegendes Polymer. Unter pflegenden Polymeren sind sowohl natürliche als auch synthetische Polymere, welche anionisch, kationisch, amphoter geladen oder nichtionisch sein können, zu verstehen.

Unter kationischen Polymeren sind Polymere zu verstehen, welche in der Haupt- und/oder Seitenkette Gruppen aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C₁₋₄-Kohlenwasserstoffgruppe an eine aus Acrylsäure, Methacrylsäure oder deren Derivaten aufgebaute Polymerhauptkette gebunden sind, haben sich als besonders geeignet erwiesen.

Bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate,
- kationische Alkylpolyglycoside gemäß der DE-PS 44 13 686,
- kationiserter Honig, beispielsweise das Handelsprodukt Honeyquat^{®} 50,
- kationische Guar-Derivate, wie insbesondere die unter den Handelsnamen Cosmedia^{®}Guar und Jaguar^{®} vertriebenen Produkte,
- Polysiloxane mit quaternären Gruppen, wie beispielsweise die im Handel erhältlichen Produkte Q2-7224 (Hersteller: Dow Corning; ein stabilisiertes Trimethylsilylamodimethicon), Dow Corning^{®} 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quatemium-80),
- polymere Dimethyldiallylammoniumsalze und deren Copolymere mit Estern und Amiden von Acrylsäure und Methacrylsäure. Die unter den Bezeichnungen Merquat^{®}100 (Poly(dimethyldiallylammoniumchlorid)) und Merquat^{®}550 (Dimethyldiallyl-ammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat^{®}734 und Gafquat^{®}755 im Handel erhältlich,
- Vinylpyrrolidon-Vinylimidazoliummethochlorid-Copolymere, wie sie unter den Bezeichnungen Luviquat^{®} FC 370, FC 550, FC 905 und HM 552 angeboten werden.
- quaternierter Polyvinylalkohol,
sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft^{®} LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix^{®} VC 713 (Hersteller: ISP), Gafquat^{®}ASCP 1011, Gafquat^{®}HS 110, Luviquat^{®}8155 und Luviquat^{®} MS 370 erhältlich sind.

Weitere erfindungsgemäße kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen^{®} CMF, Hydagen^{®} HCMF, Kytamer^{®} PC und Chitolam^{®} NB/101 im Handel frei verfügbar sind. Chitosane sind deacetylierte Chitine, die in unterschiedlichen Deacetylierungsgraden und unterschiedlichen Abbaugraden (Molekulargewichten) im Handel erhältlich sind. Ihre Herstellung ist z.B. in DE 44 40 625 A1 und in DE 1 95 03 465 A1 beschrieben.

Besonders gut geeignete Chitosane weisen einen Deacetylierungsgrad von wenigstens 80 % und ein Molekulargewicht von 5 · 10⁵ bis 5 · 10⁶ (g/mol) auf.

Zur Herstellung erfindungsgemäßer Zubereitungen muß das Chitosan in die Salzform überführt werden. Dies kann durch Auflösen in verdünnten wäßrigen Säuren erfolgen. Als Säuren sind sowohl Mineralsäuren wie z.B. Salzsäure, Schwefelsäure und Phosphorsäure als auch organische Säuren, z.B. niedermolekulare Carbonsäuren, Polycarbonsäuren und Hydroxycarbonsäuren geeignet. Weiterhin können auch höhermolekulare Alkylsulfonsäuren oder Alkylschwefelsäuren oder Organophosphorsäuren verwendet werden, soweit diese die erforderliche physiologische Verträglichkeit aufweisen. Geeignete Säuren zur Überführung des Chitosans in die Salzform sind z.B. Essigsäure, Glycolsäure, Weinsäure, Apfelsäure, Citronensäure, Milchsäure, 2-Pyrrolidinon-5-carbonsäure, Benzoesäure oder Salicylsäure. Bevorzugt werden niedermolekulare Hydroxycarbonsäuren wie z.B. Glycolsäure oder Milchsäure verwendet.

Weiterhin können als Polymere zur Steigerung der Wirkung des erfindungsgemäßen Wirkstoffes amphotere Polymere als Bestandteil eingesetzt. Unter dem Begriff amphotere Polymere werden sowohl solche Polymere, die im Molekül sowohl freie Aminogruppen als auch freie -COOH- oder SO₃H-Gruppen enthalten und zur Ausbildung innerer Salze befähigt sind, als auch zwitterionische Polymere, die im Molekül quartäre Ammoniumgruppen und -COO⁻- oder -SO₃⁻-Gruppen enthalten, und solche Polymere zusammengefaßt, die -COOH- oder SO₃H-Gruppen und quartäre Ammoniumgruppen enthalten.

Ein Beispiel für ein erfindungsgemäß einsetzbares Amphopolymer ist das unter der Bezeichnung Amphomer^{®} erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt.

Ein weiteres Beispiel für ein erfindungsgemäß einsetzbares Polymer ist das beispeielsweise unter der Bezeichnung Merquat^{®} 280 erhältliche Polyquatemium-22, das ein Copolymeres aus Dimethyldiallylammoniumchlorid und Acrylsäure darstellt.

Weitere erfindungsgemäß einsetzbare amphotere Polymere sind die in der britischen Offenlegungsschrift 2 104 091, der europäischen Offenlegungsschrift 47 714, der europäischen Offenlegungsschrift 217 274, der europäischen Offenlegungsschrift 283 817 und der deutschen Offenlegungsschrift 28 17 369 genannten Verbindungen.

Bevorzugt eingesetzte amphotere Polymere sind solche Polymerisate, die sich im wesentlichen zusammensetzen aus
(a) Monomeren mit quartären Ammoniumgruppen der allgemeinen Formel (VII),

   R²²-CH=CR²³-CO-Z-(CₙH₂ₙ)-N⁽⁺⁾R²⁴R²⁵R²⁶ A⁽⁻⁾ (VII)

   in der R²² und R²³ unabhängig voneinander stehen für Wasserstoff oder eine Methylgruppe und R²⁴, R²⁵ und R²⁶ unabhängig voneinander für Alkylgruppen mit 1 bis 4 Kohlenstoff-Atomen, Z eine NH-Gruppe oder ein Sauerstoffatom, n eine ganze Zahl von 2 bis 5 und A⁽⁻⁾ das Anion einer organischen oder anorganischen Säure ist und
(b) monomeren Carbonsäuren der allgemeinen Formel (VIII),

   R²⁷-CH=CR²⁸-COOH (VII)
in denen R²⁷ und R²⁸ unabhängig voneinander Wasserstoff oder Methylgruppen sind.

Diese Verbindungen können sowohl direkt als auch in Salzform, die durch Neutralisation der Polymerisate, beispielsweise mit einem Alkalihydroxid, erhalten wird, erfindungsgemäß eingesetzt werden. Bezüglich der Einzelheiten der Herstellung dieser Polymerisate wird ausdrücklich auf den Inhalt der deutschen Offenlegungsschrift 39 29 973 Bezug genommen. Ganz besonders bevorzugt sind solche Polymerisate, bei denen Monomere des Typs (a) eingesetzt werden, bei denen R²⁴, R²⁵ und R²⁶ Methylgruppen sind, Z eine NH-Gruppe und A⁽⁻⁾ ein Halogenid-, Methoxysulfat- oder Ethoxysulfat-Ion ist; Acrylamidopropyl-trimethyl-ammoniumchlorid ist ein besonders bevorzugtes Monomeres (a). Als Monomeres (b) für die genannten Polymerisate wird bevorzugt Acrylsäure verwendet.

Ein bevorzugtes derartiges Polymer ist beispeielsweise das unter der Bezeichnung Merquat^{®} 2001 von der Firma Calgon vertriebene Polyquaternium-47.

Die erfindungsgemäßen Mittel können in einer dritten Variante weiterhin nichtionogene pflegende Polymere enthalten.

Geeignete nichtionogene Polymere sind beispielsweise:
- Schellack
- Siloxane. Diese Siloxane können sowohl wasserlöslich als auch wasserunlöslich sein. Geeignet sind sowohl flüchtige als auch nichtflüchtige Siloxane, wobei als nichtflüchtige Siloxane solche Verbindungen verstanden werden, deren Siedepunkt bei Normaldruck oberhalb von 200 °C liegt. Bevorzugte Siloxane sind Polydialkylsiloxane, wie beispielsweise Polydimethylsiloxan, Polyalkylarylsiloxane, wie beispielsweise Polyphenylmethylsiloxan, ethoxylierte Polydialkylsiloxane sowie Polydialkylsiloxane, die Amin- und/oder Hydroxy-Gruppen enthalten.
- Glycosidisch substituierte Silicone gemäß der EP 0612759 B1.

Es ist erfindungsgemäß auch möglich, daß die verwendeten Zubereitungen mehrere, insbesondere zwei verschiedene pflegende Polymere gleicher Ladung und/oder jeweils ein ionisches und ein amphoteres und/oder nicht ionisches Polymer enthalten.

Unter dem Begriff pflegendes Polymer sind erfindungsgemäß ebenfalls spezielle Zubereitungen von Polymeren wie sphärische Polymerpulver zu verstehen. Es sind verschiedene Verfahren bekannt, solche Mikrokugeln aus verschiedenen Monomeren herzustellen, z.B. durch spezielle Polymerisationsverfahren oder durch Auflösen des Polymeren in einem Lösungsmittel und Versprühen in ein Medium, in dem das Lösungsmittel verdunsten oder aus den Teilchen herausdiffundieren kann. Ein solches Verfahren ist z.B. aus EP 466 986 B1 bekannt. Geeignete Polymerisate sind z.B. Polycarbonate, Polyurethane, Polyacrylate, Polyolefine, Polyester oder Polyamide. Besonders geeignet sind solche sphärischen Polymerpulver, deren Primärpartikeldurchmesser unter 1 µm liegt. Solche Produkte auf Basis eines Polymethacrylat-Copolymers sind z.B. unter dem Warenzeichen Polytrap^{®}Q5-6603 (Dow Corning) im Handel. Andere Polymerpulver, z.B. auf Basis von Polyamiden (Nylon 6, Nylon 12) sind mit einer Teilchengröße von 2-10 µm (90 %) und einer spezifischen Oberfläche von ca. 10 m²/g unter der Handelsbezeichnung Orgasol^{®} 2002 DU Nat Cos (Atochem S.A., Paris) erhältlich. Weitere sphärische Polymerpulver, die für den erfindungsgemäßen Zweck geeignet sind, sind z.B. die Polymethacrylate (Micropearl M) von SEPPIC oder (Plastic Powder A) von NIKKOL, die Styrol-Divinylbenzol-Copolymeren (Plastic Powder FP) von NIKKOL, die Polyethylen- und Polypropylen-Pulver (ACCUREL EP 400) von AKZO, oder auch Silikonpolymere (Silicone Powder X2-1605) von Dow Corning oder auch sphärische Cellulosepulver.

Die pflegenden Polymere sind in den erfindungsgemäß verwendeten Mitteln bevorzugt in Mengen von 0,01 bis 10 Gew.-%, bezogen auf das gesamte Mittel, enthalten. Mengen von 0,1 bis 5, insbesondere von 0,1 bis 2 Gew.-%, sind besonders bevorzugt.

### Weitere Bestandteile

Zur Herstellung der erfindungsgemäßen Färbemittel können die Farbstoffvorprodukte in einen geeigneten wasserhaltigen Träger eingearbeitet werden. Zum Zwecke der Haarfärbung sind solche Träger z.B. Cremes, Emulsionen, Gele oder auch tensidhaltige schäumende Lösungen, z.B. Shampoos, Schaumaerosole oder andere Zubereitungen, die für die Anwendung auf dem Haar geeignet sind.

Die erfindungsgemäßen Färbemittel können weiterhin alle für solche Zubereitungen bekannten Wirk-, Zusatz- und Hilfsstoffe enthalten. In vielen Fällen enthalten die Färbemittel mindestens ein weiteres Tensid, wobei prinzipiell sowohl anionische als auch zwitterionische, ampholytische, nichtionische und kationische Tenside geeignet sind. Der Fachmann kann einen eventuellen Einfluß der verschiedenen Tenside auf die Aktivität des erfindungsgemäßen Enzymsystems gegebenenfalls durch einfache Vorversuche überprüfen.

In einer bevorzugten Ausführungsform der vorliegenden Erfindung wird in den Mitteln zur Färbung keratinischer Fasern eine Kombination aus anionischen und nichtionischen Tensiden oder eine Kombination aus anionischen und amphoteren Tensiden eingesetzt.

Es hat sich aber in Einzelfällen als vorteilhaft erwiesen, die Tenside aus amphoteren oder nichtionischen Tensiden auszuwählen, da diese in der Regel den erfindungsgemäßen Färbeprozeß weniger beeinflussen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)₂-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Eiweißsäurekonzentrate,
- Cocosmonoglyceridsulfate,
- Amidethersulfate.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethyl-ammoniumglycinate, beispielsweise das Kokosacylaminopropyl-dimethylammonium-glycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Erfindungsgemäß werden als kationische Tenside insbesondere solche vom Typ der quartären Ammoniumverbindungen, der Esterquats und der Amidoamine eingesetzt.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyl-dimethylamin dar.

Weitere erfindungsgemäß verwendbare kationische Tenside stellen die quaternisierten Proteinhydrolysate dar.
Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).
Ein Beispiel für ein als kationisches Tensid einsetzbares quaternäres Zuckerderivat stellt das Handelsprodukt Glucquat^{®}100 dar, gemäß INCI-Nomenklatur ein "Lauryl Methyl Gluceth-10 Hydroxypropyl Dimonium Chloride".

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weiterhin enthalten die erfindungsgemäßen Mittel bevorzugt mindestens ein Alkalisierungsmittel. Bevorzugte Alkalisierungsmittel sind Ammoniak, Monoethanolamin, Isopropylamin, Isopropanolamin, 2-Amino-2-methylpropan-1-ol, ein Alkalimetallhydroxid, insbesondere Natriumhydroxid oder Kaliumhydroxid, und insbesondere Arginin, Lysin und Histidin. Erfindungsgemäß besonders bevorzugt sind Färbemittel, die Monoethanolamin, 2-Amino-2-methylpropan-1-ol, eine Alkalimetallhydroxid, insbesondere Natriumhydroxid oder Kaliumhydroxid, und insbesondere Arginin, Lysin und Histidin enthalten.

Weiterhin können die erfindungsgemäßen Färbemittel bevorzugt noch einen konditionierenden Wirkstoff, ausgewählt aus der Gruppe, die von kationischen Tensiden, Alkylamidoaminen, Paraffinölen, pflanzlichen Ölen und synthetischen Ölen gebildet wird, enthalten. Hinsichtlich der kationischen Tenside sei auf die obigen Ausführungen verwiesen.

Als konditionierende Wirkstoffe weiterhin geeignet sind Silikonöle, insbesondere Dialkyl- und Alkylarylsiloxane, wie beispielsweise Dimethylpolysiloxan und Methylphenylpolysiloxan, sowie deren alkoxylierte und quaternierte Analoga. Beispiele für solche Silikone sind die von Dow Corning unter den Bezeichnungen DC 190, DC 200, DC 344, DC 345 und DC 1401 vertriebenen Produkte sowie die Handelsprodukte Q2-7224 (Hersteller: Dow Corning; ein stabilisiertes Trimethylsilylamodimethicon), Dow Corning^{®} 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).

Ebenfalls einsetzbar als konditionierende Wirkstoffe sind Paraffinöle, synthetisch hergestellte oligomere Alkene sowie pflanzliche Öle wie Jojobaöl, Sonnenblumenöl, Orangenöl, Mandelöl, Weizenkeimöl und Pfirsichkernöl.

Gleichfalls geeignete haarkonditionierende Verbindungen sind Phospholipide, beispielsweise Sojalecithin, Ei-Lecithin und Kephaline.

Weiterhin enthalten die erfindungsgemäß verwendeten Zubereitungen bevorzugt mindestens eine Ölkomponente.

Erfindungsgemäß geeignete Ölkomponenten sind prinzipiell alle wasserunlöslichen Öle und Fettstoffe sowie deren Mischungen mit festen Paraffinen und Wachsen. Als wasserunlöslich werden erfindungsgemäß solche Stoffe definiert, deren Löslichkeit in Wasser bei 20 °C kleiner als 0,1 Gew.-% beträgt. Der Schmelzpunkt der einzelnen Öl- oder Fettkomponenten liegt bevorzugt unterhalb von etwa 40 °C. Öl- und Fettkomponenten, die bei Raumtemperatur, d. h. unterhalb von 25 °C flüssig sind, können erfindungsgemäß besonders bevorzugt sein. Bei Verwendung mehrerer Öl- und Fettkomponenten sowie ggf. festen Paraffinen und Wachsen ist es in der Regel jedoch auch ausreichend, wenn die Mischung der Öl- und Fettkomponenten sowie ggf. Paraffine und Wachse diesen Bedingungen genügt.

Eine bevorzugte Gruppe von Ölkomponenten sind pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls.

Geeignet sind aber auch andere Triglyceridöle wie die flüssigen Anteile des Rindertalgs sowie synthetische Triglyceridöle.

Eine weitere, besonders bevorzugte Gruppe erfindungsgemäß als Ölkomponente einsetzbarer Verbindungen sind flüssige Paraffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol^{®} S) und Di-n-octylether (Cetiol^{®} OE) können bevorzugt sein.

Ebenfalls erfindungsgemäß einsetzbare Ölkomponenten sind Fettsäure- und Fettalkoholester. Bevorzugt sind die Monoester der Fettsäuren mit Alkoholen mit 3 bis 24 C-Atomen. Bei dieser Stoffgruppe handelt es sich um die Produkte der Veresterung von Fettsäuren mit 6 bis 24 C-Atomen wie beispielsweise Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z. B. bei der Druckspaltung von natürlichen Fetten und Ölen, bei der Oxidation von Aldehyden aus der Roelen'schen Oxosynthese oder der Dimerisierung von ungesättigten Fettsäuren anfallen, mit Alkoholen wie beispielsweise Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmitoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z. B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat, Isononansäure-C16-18-alkylester (Cetiol^{®} SN), Stearinsäure-2-ethylhexylester (Cetiol^{®} 868), Cetyloleat, Glycerintricaprylat, Kokosfettalkohol-caprinat/-caprylat und n-Butylstearat.

Weiterhin stellen auch Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-di-isotridecanoat. Propylenglykol-di(2-ethylhexanoat), Propylenglykol-di-isostearat, Propylenglykol-di-pelargonat, Butandiol-di-isostearat und Neopentylglykoldi-capylat erfindungsgemäß verwendbare Ölkomponenten dar, ebenso komplexe Ester wie z. B. das Diacetyl- glycerinmonostearat.

Schließlich können auch Fettalkohole mit 8 bis 22 C-Atomen als erfindungsgemäß wirkende Ölkomponenten eingesetzt werden. Die Fettalkohole können gesättigt oder ungesättigt und linear oder verzweigt sein. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen.

Die Ölkomponenten werden bevorzugt in Menden von 0,05 bis 10 Gew.-%, insbesondere von 0,1 bis 2 Gew.-% in den erfindungsgemäßen Färbemitteln eingesetzt.

Weitere Wirk-, Hilfs- und Zusatzstoffe sind beispielsweise
- Strukturanten wie Maleinsäure und Milchsäure,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1 -alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, B₃, B₅, B₆, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wasserstoffperoxid und andere Oxidationsmittel,
- Treibmittel wie Propan-Butan-Gemische, N₂O, Dimethylether, CO₂ und Luft,
- Antioxidantien und
- Konservierungsmittel.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Zweckmäßigerweise wird die Enzymzubereitung unmittelbar vor dem Färben des Haar mit der Zubereitung aus den Farbstoffvorprodukten vermischt. Das dabei entstehende gebrauchsfertige Haarfärbepräparat sollte bevorzugt einen pH-Wert im Bereich von 4 bis 10, insbesondere von 6 bis 9, ganz besonders von 7 bis 8,5 aufweisen. Ferner haben sich niedrigviskose Anwendungszubereitungen als besonders vorteilhaft erwiesen, die eine Viskosität von weniger als 3000mPas (Brookfield, Spindel 4, 20 U/min, 20°C) aufweisen. Die Anwendungstemperaturen können in einem Bereich zwischen 10 und 50°C, vorzugsweise zwischen 20 und 35°C, liegen. Nach einer Einwirkungszeit von ca. 30 Minuten wird das Haarfärbemittel durch Ausspülen von dem zu färbenden Haar entfernt. Das Nachwaschen mit einem Shampoo entfällt, wenn ein stark tensidhaltiger Träger, z. B. ein Färbeshampoo, verwendet wurde.

Um die Färbeleistung der erfindungsgemäßen Mittel weiter zu erhöhen, kann es erfindungsgemäß bevorzugt sein, die Fasern nach der Behandlung mit dem erfindungsgemäßen Mittel einer Nachbehandlung mit einer Lösung, die geringe Mengen Wasserstoffperoxid enthält, zu unterziehen. Die Lösung kann beispielsweise in Form eines Shampoos oder eines Konditioniermittels konfektioniert sein. Üblicherweise enthalten derartige Nachbehandlungsmittel Wasserstoffperoxid in Konzentrationen zwischen 0,01 und 0,5 Gew.-%.

In einer weiteren Ausführungsform der vorliegenden Erfindung kann es bevorzugt sein, die Mittel, insbesondere die separat konfektionierte Enzymzubereitung, frei von Antioxidantien und/oder Komplexbildnern zu formulieren, da diese die Wirkung der Enzyme blockieren können.

Ein zweiter Gegenstand der vorliegenden Erfindung ist ein Verfahren zum Färben keratinischer Fasern, bei dem ein erfindungsgemäßes Mittel auf die Fasern aufgetragen wird und nach einer Einwirkungszeit wieder abgespült wird.

Ein dritter Gegenstand der vorliegenden Erfindung ist ein Verfahren zum Färben keratinischer Fasern, bei dem ein erfindungsgemäßes Mittel angewendet wird, wobei in einem ersten Schritt eine Zubereitung, enthaltend die Komponenten (A), (C) und (D) auf die Fasern aufgetragen wird, die Fasern nach eine Einwirkzeit gegebenenfalls gespült werden und in einem zweiten Schritt eine Zubereitung, enthaltend die Komponente (B), auf die Fasern aufgetragen wird. Ebenfalls als erfindungsgemäß geeignet hat sich ein Verfahren erwiesen, bei dem in einem ersten Schritt die Fasern mit einer Zubereitung enthaltend die Komponente (B) behandelt werden, die Fasern nach einer Einwirkungszeit gegebenenfalls gespült werden und anschließend eine Zubereitung, enthaltend die Komponenten (A), (C) und (D), aufgetragen wird.

Ein vierter Gegenstand der vorliegenden Erfindung ist die Verwendung der erfindungsgemäßen Mittel zur oxidativen Färbung keratinischer Fasern.

Ein fünfter Gegenstand der vorliegenden Erfindung ist ein Kit zum Färben keratinischer Fasern, umfassend zumindest eine erste Zubereitung, enthaltend in einem kosmetisch akzeptablen Träger
(A) mindestens ein Farbstoffvorprodukt,
(C) mindestens ein Zuckertensid, ausgewählt aus der Gruppe, die gebildet wird von
   - Alkyl- und Alkenyloligoglykosiden und
   - Fettsäure-N-alkyl-polyhydroxyalkylamiden und
(D) mindestens ein Reduktionsmittel,
   und eine zweite Zubereitung, enthaltend in einem kosmetisch akzeptablen Träger
(B) mindestens ein Enzym, das die Oxidation der Farbstoffvorprodukte katalysieren kann ausgewählt aus den Laccasen (EC-No. 1.10.3.2), den Tyrosinasen (EC-No. 1.10.3.1), den Ascorbat Oxidase (EC-N°. 1.10.3.3), den Bilirubin-Oxidasen (EC-Nr.1.3.35) sowie den Phenoloxidasen vom Typ Acremonia, Stachybotrys oder Pleurotus.

Die folgenden Beispiele sollen die Erfindung näher erläutern:

### Beispiele

Die Mengenangaben in den folgenden Beispielen sind, soweit nicht anders vermerkt, Gewichtsprozent.

### I) Zusammensetzung der Färbegele

### Färbegel 1:

| | |
|---|---|
| Natrosol^{®} 250 HR¹ | 2.0 |
| Eumulgin^{®} B2² | 0.5 |
| Plantacare^{®} 1200 UP³ | 0.5 |
| Lamesoft^{®} PO65⁴ | 0.2 |
| Tri-Natriumcitrat-dihydrat | 0.5 |
| 2,4,5,6-Tetraaminopyrimidin-sulfat | 0.1 |
| p-Toluylendiamin-sulfat | 0.1 |
| 4-Amino-3-methylphenol | 0.25 |
| m-Aminophenol | 0.1 |
| 4-Chlorresorcin | 0.12 |
| 2-Amino-3-hydroxypyridin | 0.13 |
| Monoethanolamin | ad pH 7 |
| Wasser | ad 100 |

| | |
|---|---|
| ¹ Hydroxyethylcellulose (INCI-Bezeichnung: Hydroxyethylcellulose) (Hercules) ² Cetylstearylalkohol mit ca. 20-EO-Einheiten (INCI-Bezeichnung: Ceteareth-20) (Cognis) ³ C₁₂₋₁₆-Alkyl-1.4-glucosid (ca. 50 bis 53 % Aktivsubstanz) INCI-Bezeichnung: Lauryl Glucoside) (Cognis) ⁴ Alkylpolyglucosid-Ölsäuremonoglycerid-Gemisch (ca. 32 bis 35 % Wassergehalt INCI-Bezeichnung: Glucoside, Glyceryl Oleate, Aqua (Water)) (Cognis) | |

### Färbegel 2:

| | |
|---|---|
| Natrosol^{®} 250 HR | 2.0 |
| Plantacare^{®} 1200 UP | 0.5 |
| Lamesoft^{®} PO65 | 0.2 |
| Polymer W 37194⁵ | 0.1 |
| Tri-Natriumcitrat-dihydrat | 0.5 |
| 2,4,5,6-Tetraaminopyrimidin-sulfat | 0.1 |
| 1-(β-Hydroxyethyl)-p-phenylendiamin-sulfat | 0.1 |
| 4-Amino-3-methylphenol | 0.2 |
| m-Aminophenol | 0.1 |
| 4-Chlorresorcin | 0.1 |
| 2-Amino-3-hydroxypyridin | 0.13 |
| Rodol 9R Base⁶ | 0.1 |
| Monoethanolamin | ad pH 7 |
| Wasser | ad 100 |

Acrylsäure-Natrium-Salz-Acrylamidopropyltrimethylammoniumchlorid-Copolymer konserviert mit Phenonip (ca. 19-21% Aktivsubstanz, INCI-Bezeichnung: Acrylamidopropyl-Trimonium Chloride / Acrylates Copolymer) (Stockhausen)
2-Amino-6-chloro-4-nitrophenol

### Färbegel 3:

| | |
|---|---|
| Natrosol^{®} 250 HR | 2.0 |
| Eumulgin^{®} B2 | 0.5 |
| Plantacare^{®} 1200 UP | 1.0 |
| Lamesoft^{®} PO65 | 0.5 |
| N-Acetyl-L-cystein | 0.15 |
| 2,4,5,6-Tetraaminopyrimidin-sulfat | 1.5 |
| p-Phenylendiamin-sulfat | 0.2 |
| 4-Amino-2-aminomethylphenol-dihydrochlorid | 0.05 |
| 2-Methylresorcin | 1.0 |
| 1-(β-Hydroxyethylamino)-4-methyl-2-nitrobenzol | 0.1 |
| L-Arginin | 1.0 |
| Monoethanolamin | ad pH 7 |
| Wasser | ad 100 |

### Färbegel 4:

| | |
|---|---|
| Natrosol^{®} 250 HR | 2.0 |
| Eumulgin^{®} B2 | 0.5 |
| Plantacare^{®} 1200 UP | 0.5 |
| Lamesoft^{®} PO65 | 0.2 |
| Tri-Natriumcitrat-dihydrat | 0.5 |
| 2,4,5,6-Tetraaminopyrimidin-sulfat | 0.2 |
| 4,5-Diamino-1-(β-hydroxyethyl)-pyrazol | 0.8 |
| 4-Amino-3-methylphenol | 0.4 |
| 2-Methylresorcin | 0.2 |
| 2,7-Dihydroxynaphthalin | 0.6 |
| 4-Chlorresorcin | 0.2 |
| Lysin | 0.2 |
| Isopropanolamin | ad pH 7 |
| Wasser | ad 100 |

### Färbegel 5:

| | |
|---|---|
| Natrosol^{®} 250 HR | 2.0 |
| Eumulgin^{®} B2 | 0.5 |
| Plantacare^{®} 1200 UP | 0.5 |
| Lamesoft^{®} PO65 | 0.2 |
| N-Acetyl-L-cystein | 0.2 |
| p-Toluylendiamin-sulfat | 1.1 |
| p-Aminophenol | 0.1 |
| 2-Methylresorcin | 0.1 |
| 2,7-Dihydroxynaphthalin | 0.3 |
| 5,6-Dihydroxyindolin-hydrobromid | 0.2 |
| m-Aminophenol | 0.06 |
| 2-Methylamino-3-amino-6-methoxypyridin | 0.02 |
| 2-Amino-3-hydroxypyridin | 0.01 |
| Resorcin | 0.2 |
| Monoethanolamin | ad pH 7 |
| Wasser | ad 100 |

### Färbegel 6:

| | |
|---|---|
| Natrosol® 250 HR | 2.0 |
| Eumulgin® B2 | 0.5 |
| Plantacare® 1200 UP | 1.5 |
| Tri-Natriumcitrat-dihydrat | 0.2 |
| N-Acetyl-L-cystein | 0.2 |
| p-Toluylendiamin-sulfat | 0.6 |
| Bis-(2-hydroxy-5-aminophenyl)-methan | 0.03 |
| 2-Methylresorcin | 0.07 |
| m-Aminophenol | 0.03 |
| 4-Chlorresorcin | 0.18 |
| Resorcin | 0.1 |
| Histidin | 0.5 |
| Monoethanolamin | ad pH 7 |
| Wasser | ad 100 |

### Färbegel 7:

| | |
|---|---|
| Carbopol^{®} 934⁷ | 1.5 |
| Eumulgin^{®} B2 | 0.5 |
| Plantacare^{®} 1200 UP | 1.5 |
| Polymer JR^{®} 400⁸ | 0.5 |
| Tri-Natriumcitrat-dihydrat | 0.5 |
| 2,4,5,6-Tetraaminopyrimidin-sulfat | 0.2 |
| p-Toluylendiamin-sulfat | 0.9 |
| 4-Amino-2-aminomethylphenol-dihydrochlorid | 0.4 |
| 2-Methylresorcin | 0.25 |
| 2,7-Dihydroxynaphthalin | 0.6 |
| 4-Chlorresorcin | 0.24 |
| 5,6-Dihydroxyindolin-hydrobromid | 0.2 |
| L-Arginin | 1.0 |
| Isopropanolamin | ad pH 7 |
| Wasser | ad 100 |

| | |
|---|---|
| ⁷ Carboxyvinylpolymer (INCI-Bezeichnung: Carbomer) (Goodrich) ⁸ quaternierte Hydroxyethylcellulose (INCI-Bezeichnung: Polyquaternium-10) (Amerchol) | |

### Färbegel 8:

| | |
|---|---|
| Carbopol^{®} 934 | 1.0 |
| Eumulgin^{®} B2 | 0.2 |
| Eumulgin^{®} B1⁹ | 0.2 |
| Plantacare^{®} 1200 UP | 0.5 |
| Lamesoft^{®} PO65 | 0.2 |
| Tri-Kaliumcitratdihydrat | 0.5 |
| 2,4,5,6-Tetraaminopyrimidin-sulfat | 0.7 |
| p-Toluylendiamin-sulfat | 0.4 |
| 2-(β-Hydroxyethyl)-p-phenylendiamin-sulfat | 0.1 |
| 3-Methyl-4-aminophenol | 0.5 |
| 2-Methylresorcin | 0.5 |
| 2,7-Dihydroxynaphthalin | 0.16 |
| 4-Chlorresorcin | 0.1 |
| 2-Amino-3-hydroxypyridin | 0.5 |
| Rodol 9R Base | 0.1 |
| Histidin | 0.2 |
| Monoethanolamin | ad pH 7 |
| Wasser | ad 100 |

| | |
|---|---|
| ⁹ Cetylstearylalkoholmit ca. 12-EO-Einheiten (INCI-Bezeichnung: Ceteareth-12) (Cognis) | |

### Färbegel 9:

| | |
|---|---|
| Natrosol^{®} 250 HR | 2.0 |
| Eumulgin^{®} B2 | 0.2 |
| Eumulgin^{®} B1 | 0.2 |
| Plantacare^{®} 1200 UP | 1.5 |
| Lamesoft^{®} PO65 | 0.2 |
| Merquat^{®} 280¹⁰ | 0.8 |
| Tri-Natriumcitrat-dihydrat | 1.0 |
| 2,4,5,6-Tetraaminopyrimidin-sulfat | 0.6 |
| p-Toluylendiamin-sulfat | 0.02 |
| 3-Methyl-4-aminophenol | 0.6 |
| 4-Chlorresorcin | 0.03 |
| 2-Amino-3-hydroxypyridin | 0.5 |
| 1-(β-Hydroxyethylamino)-4-methyl-2-nitrobenzol | 0.1 |
| Isopropanolamin | ad pH 7 |
| Wasser | ad 100 |

| | |
|---|---|
| ¹⁰ Dimethyldiallylammoniumchlorid-Acrylsäure-Copolymer (ca. 35% Aktivsubstanz, INCI-Bezeichnung: Polyquaternium-22) (Chemviron) | |

### Färbegel 10:

| | |
|---|---|
| Aculyn^{®} 22¹¹ | 2.0 |
| Eumulgin^{®} B2 | 0.5 |
| Plantacare^{®} 1200 UP | 0.5 |
| Lamesoft^{®} PO65 | 0.2 |
| Tri-Natriumcitrat-dihydrat | 0.5 |
| Tri-Kaliumcitrat-dihydrat | 0.5 |
| 2,4,5,6-Tetraaminopyrimidin-sulfat | 0.6 |
| p-Toluylendiamin-sulfat | 0.27 |
| 2,6-Di-(β-hydroxyethylamino)-toluol | 0.4 |
| 3,5-Diamino-2,6-dimethoxypyridin | 0.5 |
| Natronlauge | ad pH 7 |
| Wasser | ad 100 |

| | |
|---|---|
| ¹¹ Methacrylsäure(stearylalkohol+20-EO-ester)-Acrylsäure-Copolymer (ca. 30% Aktivsubstanz, INCI-Bezeichnung: Acrylates/Steareth-20 Methacrylate Copolymer) (Rohm & Haas) | |

### Färbegel 11:

| | |
|---|---|
| Natrosol^{®} 250 HR | 3.0 |
| Eumulgin^{®} B2 | 0.5 |
| Plantacare^{®} 1200 UP | 1.5 |
| Lamesoft^{®} PO65 | 0.2 |
| Tri-Natriumcitrat-dihydrat | 0.5 |
| p-Toluylendiamin-sulfat | 1.1 |
| 1,3-Bis-(2',4'-diaminophenoxy)-propan-hydrochlorid | 0.4 |
| m-Aminophenol | 0.1 |
| 2-Methylamino-3-amino-6-methoxypyridin | 0.2 |
| 2-Amino-4-(β-hydroxyethylamino)-anisol-sulfat | 0.2 |
| Monoethanolamin | ad pH 7 |
| Wasser | ad 100 |

### II) Enzyme

### a) Laccase

Es wurde eine Laccase aus *Rhus vernificera* der Firma Sigma verwendet. Die Aktivität der Laccase ist gemäß den Herstellerangaben derart definiert, daß eine Einheit [1U] der Menge Laccase entspricht, die ein ΔA₅₃₀ von 0,001 pro Minute bei einem pH-Wert von 6,5 und 30°C in 3ml einer Reaktionslösung mit Syringaldazin als Substrat ergibt.
Die Laccase wurde jeweils in einer solchen Menge eingesetzt, die unter den oben genannten Bedingungen eine Aktivität von 280 000U aufweist.

### b) Ascorbatoxidase

Es wurde eine Ascorbat-Oxidase aus *Cucurbita species* der Firma Roche-Diagnostics verwendet. Die Aktivität der Ascorbat-Oxidase ist derart definiert, daß eine Einheit [1U] der Menge Ascorbat-Oxidase entspricht, die in einer Phosphatpuffer-Lösung (KH₂PO₄, 0,1 mol/l; Na₂HPO₄, 4mmol/l; EDTA, 0,5mmol/l) die Oxidation von 1µmol L-Ascorbinsäure innerhalb einer Minute bei pH 5,6 und 25°C katalysiert (entsprechend der Definition der Handelsprodukte der Firma Roche Diagnostics). Die Detektion erfolgt spektroskopisch anhand der Abnahme der Absorption bei 245nm. Die Ascorbat-Oxidase wurde jeweils in einer solchen Menge eingesetzt, die unter den oben genannten Bedingungen eine Aktivität von 100 000U aufweist.

### c) Bilirubinoxidase

Es wurde eine Bilirubinoxidase aus *Myrothecium verrucaria* der Fa. Sigma eingesetzt. Die Aktivität der Bilirubinoxidase ist derart definiert, daß eine Einheit [1U] der Menge an Bilirubinoxidase entspricht, die die Oxidation von 1 µmol Bilirubin innerhalb einer Minute bei pH 8,4 und 37°C katalysiert.
Die Bilirubinoxidase wurde jeweils in einer solchen Menge eingesetzt, die unter den oben genannten Bedingungen eine Aktivität von 1 000U aufweist.

### III) Ausfärbung

### Enzyme a bis c

60g des jeweiligen Färbegels 1 bis 11 wurden mit 10 ml einer (durch KHPO₄/KH₂PO₄) auf pH 7 gepufferten Lösung, die die Enzyme a-c in den unter Punkt II) genannten Aktivitäten enthält, vermischt. Die Mischung wurde auf Humanhaar aufgebracht (Kerling, naturweiß, 4g Färbemittel pro 0.5g Haar), eingearbeitet und für 30 min. bei Raumtemperatur belassen. Anschließend wurde das Haar ausgespült und getrocknet.

Die jeweiligen Färbeergebnisse sind der folgenden Tabelle zu entnehmen:

| | Farbton | | |
|---|---|---|---|
| | Laccase | Ascorbatoxidase | Bilirubinoxidase |
| Färbegel 1 | rehbraun | herbstbraun | herbstbraun |
| Färbegel 2 | herbstrot | rotbraun | herbstrot |
| Färbegel 3 | granatrot | granatrot | granatrot |
| Färbegel 4 | mittelbraun | hellbraun | mittelbraun |
| Färbegel 5 | dunkelbraun | mittel-dunkelbraun | mittel-dunkelbraun |
| Färbegel 6 | hellgoldbraun | goldbraun | goldbraun |
| Färbegel 7 | haselnußbraun | gelbbraun | gelbbraun |
| Färbegel 8 | rotkupfer | kupferrot | kupfergold |
| Färbegel 9 | kupfer | kupferbraun | kupfergold 1 |
| Färbegel 10 | dunkelviolett | dunkelviolett | dunkelviolett |
| Färbegel 11 | schwarz | schwarz | schwarz |

## Patentansprüche

1. Mittel zum Färben keratinischer Fasern enthaltend in einem kosmetisch akzeptablen Träger
(A) mindestens ein Farbstoffvorprodukt,
(B) mindestens ein Enzym, das die Oxidation der Farbstoffvorprodukte katalysieren kann, ausgewählt aus den Laccasen (EC-Nr. 1.10.3.2), den Tyrosinasen (EC-Nr. 1.10.3.1), den Ascorbat-Oxidase (EC-Nr. 1.10 3.3), den Bilirubin-Oxidasen (EC-Nr. 1.3. 3.5) sowie den Phenoloxidasen vom Typ Acremonia, Stachybotrys oder Pleurotus.
(C) mindestens ein Zuckertensid, ausgewählt aus der Gruppe, die gebildet wird von
- Alkyl- und Alkenyloligoglykosiden und
- Fettsäure-N-alkyl-polyhydroxyalkylamiden und
(D) mindestens ein Reduktionsmittel.

2. Mittel nach einem der Ansprüche 1, **dadurch gekennzeichnet, daß** es als Farbstoffvorprodukt (A) mindestens eine Entwicklerkomponente enthält

3. Mittel nach einem der Ansprüche 1 oder 2, **dadurch gekennzeichnet, daß** es als Farbstoffvorprodukt (A) mindestens ein Indol- und/oder Indolinderivat enthält.

4. Mittel nach einem der Ansprüche 1 bis 3, **dadurch gekennzeichnet, daß** es mindestens eine Kupplerkomponente enthält.

5. Mittel nach einem der Ansprüche 1 bis 4, **dadurch gekennzeichnet, daß** es weiterhin mindestens einen direktziehenden Farbstoff enthält.

6. Mittel nach einem der Ansprüche 1 bis 5, **dadurch gekennzeichnet, daß** es als Reduktionsmittel ein Alkalimetallcitratsalz, insbesondere Natriumcitrat, und/oder N-Acetyl-L-Cystein enthält.

7. Mittel nach Anspruch 6, **dadurch gekennzeichnet, daß** es als Reduktionsmittel N-Acetyl-L-Cystein enthält.

8. Mittel nach einem der Ansprüche 1 bis 7, **dadurch gekennzeichnet, daß** es weiterhin mindestens ein Fettsäurepartialglycerid enthält.

9. Mittel nach einem der Ansprüche 1 bis 8, **dadurch gekennzeichnet, daß** es weiterhin ein mindestens ein Verdickungsmittel enthält.

10. Mittel nach Anspruch 9, **dadurch gekennzeichnet, daß** das Verdickungsmittel ein anionisches oder nichtionisches Polymer ist.

11. Mittel nach einem der Ansprüche 9 oder 10, **dadurch gekennzeichnet, daß** es als. Verdickungsmittel mindestens eine Cellulose und/oder ein Cellulosederivat enthält.

12. Mittel nach einem der Ansprüche 1 bis 11, **dadurch gekennzeichnet, daß** es weiterhin ein pflegendes Polymer enthält.

13. Mittel nach einem der Ansprüche 1 bis 12, **dadurch gekennzeichnet, daß** es mindestens ein weiteres Tensid enthält

14. Mittel nach Anspruch 13, **dadurch gekennzeichnet, daß** es ein nichtionisches oder ein amphoteres Tensid enthält.

15. Mittel nach einem der Ansprüche 1 bis 14, **dadurch gekennzeichnet, daß** es mindestens ein Alkalisierungsmittel enthält.

16. Mittel nach Anspruch 15, **dadurch gekennzeichnet, daß** es als Alkalisierungsmittel Monoethanolamin, Isopropylamin, Isopropanolamin, 2-Amino-2-methylpropan-1-ol, Arginin, Histidin oder ein Alkalimetallhydroxid, insbesondere Natriumhydroxid oder Kaliumhydroxid, enthält.

17. Mittel nach einem der Ansprüche 1 bis 16, **dadurch gekennzeichnet, daß** es einen pH-Wert von 4 bis 10, insbesondere von 6 bis 9, ganz besonders bevorzugt von 7 bis 8,5, aufweist.

18. Mittel nach einem der Ansprüche 1 bis 17, **dadurch gekennzeichnet, daß** es eine niedrigviskose Zubereitung mit einer Viskosität von weniger als 3000mPas (Brookfield, Spindel 4, 20 U/min, 20°C) ist.

19. Verfahren zum Färben keratinischer Fasern, bei dem ein Mittel nach einem der Ansprüche 1 bis 18 auf die Fasern aufgetragen wird und nach einer Einwirkungszeit wieder abgespült wird.

20. Verfahren zum Färben keratinischer Fasern, bei dem ein Mittel nach einem der Anspruche 1 bis 18 augewendet wird, wobei in einem ersten Schritt eine Zubereitung, enthaltend die Komponenten (A), (C) und (D) auf die Fasern aufgetragen wird, die Fasern nach eine Einwirkungszeit gegebenenfalls gespült werden und in einem zweiten Schritt eine Zubereitung, enthaltend die Komponente (B), auf die Fasern aufgetragen wird.

21. Verwendung eines der Mittel der Ansprüche 1 bis 18 zur Färbung keratinischer Fasern.

22. Kit zum Färben keratinischer Fasern, umfassend zumindest eine erste Zubereitung, enthaltend in einem kosmetisch akzeptablen Träger
(A) mindestens ein Farbstoffvorprodukt,
(C) mindestens ein Zuckertensid, ausgewählt aus der Gruppe, die gebildet wird von
- Alkyl- und Alkenyloligoglykosiden und
- Fettsäure-N-alkyl-polyhydroxyalkylamiden und
(D) mindestens ein Reduktionsmittel,
und eine zweite Zubereitung, enthaltend in einem kosmetisch akzeptablen Träger
(B) mindestens ein Enzym, das die Oxidation der Farbstoffvorprodukte katalysieren kann ausgewählt aus den Laccasen (EC-Nr. 1.10.3.2), den Tyrosinasen (EC-Nr. 1.10.3.1), den Ascorbat-Oxidase (EC-Nr. 1.10.3.3), den Bilirubin-Oxidasen (EC-Nr. 1.3.3.5) sowie den Phenoloxidasen vom Typ Acremonia, Stachybotrys oder Pleurotus.

## Claims

1. Agent for the colouring of keratin fibres comprising, in a cosmetically acceptable carrier,
(A) at least one dye precursor,
(B) at least one enzyme which can catalyse the oxidation of the dye precursors, selected from the laccases (EC No. 1.10.3.2), the tyrosinases (EC-No.1.10.3.1), the ascorbate oxidase (EC No. 1.10.3.3), the bilirubin oxidases (EC No.1.3.3.5) and the phenol oxidases of the Acremonia, Stachybotrys or Pleurotus type,
(C) at least one sugar surfactant selected from the group which is formed by
- alkyl and alkenyl oligoglycosides and
- fatty acid N-alkylpolyhydroxyalkylamides and
(D) at least one reducing agent.

2. Agent according to Claim 1, **characterized in that** it comprises at least one developer component as dye precursor (A).

3. Agent according to one of Claims 1 and 2, **characterized in that** it comprises at least one indole and/or indoline derivative as dye precursor (A).

4. Agent according to one of Claims 1 to 3, **characterized in that** it comprises at least one coupler component.

5. Agent according to one of Claims 1 to 4, **characterized in that** it further comprises at least one direct dye.

6. Agent according to one of Claims 1 to 5, **characterized in that** it comprises an alkali metal citrate salt, in particular sodium citrate, and/or N-acetyl-L-cysteine as reducing agent.

7. Agent according to Claim 6, **characterized in that** it comprises N-acetyl-L-cysteine as reducing agent.

8. Agent according to one of Claims 1 to 7, **characterized in that** it further comprises at least one fatty acid partial glyceride.

9. Agent according to one of Claims 1 to 8, **characterized in that** it further comprises at least one thickener.

10. Agent according to Claim 9, **characterized in that** the thickener is an anionic or nonionic polymer.

11. Agent according to one of Claims 9 and 10, **characterized in that** it comprises at least one cellulose and/or one cellulose derivative as thickener.

12. Agent according to one of Claims 1 to 11, **characterized in that** it further comprises a care polymer.

13. Agent according to one of Claims 1 to 12, **characterized in that** it comprises at least one further surfactant.

14. Agent according to Claim 13, **characterized in that** it comprises one nonionic or one amphoteric surfactant.

15. Agent according to one of Claims 1 to 14, **characterized in that** it comprises at least one alkalizing agent.

16. Agent according to Claim 15, **characterized in that** it comprises monoethanolamine, isopropylamine, isopropanolamine, 2-amino-2-methylpropan-1-ol, arginine, histidine or an alkali metal hydroxide, in particular sodium hydroxide or potassium hydroxide, as alkalizing agent.

17. Agent according to one of Claims 1 to 16, **characterized in that** it has a pH of from 4 to 10, in particular from 6 to 9, very particularly preferably from 7 to 8.5.

18. Agent according to one of Claims 1 to 17, **characterized in that** it is a low-viscosity preparation with a viscosity of less than 3000 mPas (Brookfield, spindle 4, 20 rpm, 20°C).

19. Method for the colouring of keratin fibres, in which an agent according to one of Claims 1 to 18 is applied to the fibres and, after a contact time, is rinsed off again.

20. Method for the colouring of keratin fibres, in which an agent according to one of Claims 1 to 18 is used, where, in a first step, a preparation comprising the components (A), (C) and (D) is applied to the fibres, the fibres are optionally rinsed after a contact time and, in a second step, a preparation comprising component (B) is applied to the fibres.

21. Use of one of the agents of Claims 1 to 18 for colouring keratin fibres.

22. Kit for the colouring of keratin fibres, comprising at least a first preparation, comprising, in a cosmetically acceptable carrier,
(A) at least one dye precursor,
(C) at least one sugar surfactant selected from the group which is formed by
- alkyl and alkenyl oligoglycosides and
- fatty acid N-alkylpolyhydroxyalkylamides and
(D) at least one reducing agent,
and a second preparation comprising, in a cosmetically acceptable carrier,
(B) at least one enzyme which can catalyse the oxidation of the dye precursors, selected from the laccases (EC No. 1.10.3.2), the tyrosinases (EC-No.1.10.3.1), the ascorbate oxidase (EC No. 1.10.3.3), the bilirubin oxidases (EC No.1.3.3.5) and the phenol oxidases of the Acremonia, Stachybotrys or Pleurotus type.

## Revendications

1. Agent pour teindre les fibres kératiniques contenant dans un véhicule acceptable sur le plan cosmétique
(A) au moins un produit précurseur de colorant
(B) au moins une enzyme, qui peut catalyser l'oxydation des produits précurseurs de colorant, choisie parmi les laccases (EC-N°1.10.3.2), les tyrosinases (EC-N°1.10.3.1), l'ascorbate oxydase (EC-N°1.10.3.3), les bilirubine oxydases (EC-N°1.3.3.5) ainsi que les phénol oxydases du type *Acremonia, Stachybotrys ou Pleurotus.*
(C) au moins un tensio-actif glucidique, choisi dans le groupe qui est formé par
- les alkyl- et alcényl-oligoglycosides et
- les N-alkyl-polyhydroxyalkylamides d'acide gras et
(D) au moins un agent réducteur.

2. Agent selon la revendication 1, **caractérisé en ce qu'**il contient en tant que produit précurseur de colorant (A) au moins un composant révélateur.

3. Agent selon l'une quelconque des revendications 1 ou 2, **caractérisé en ce qu'**il contient en tant que produit précurseur de colorant (A) au moins un dérivé indole et/ou indoline.

4. Agent selon l'une quelconque des revendications 1 à 3, **caractérisé en ce qu'**il contient au moins un composant de couplage.

5. Agent selon l'une quelconque des revendications 1 à 4, **caractérisé en ce qu'**il contient en plus au moins un colorant montant directement sur les fibres.

6. Agent selon l'une quelconque des revendications 1 à 5, **caractérisé en ce qu'**il contient en tant qu'agent réducteur, un sel citrate de métal alcalin, en particulier le citrate de sodium, et/ou la N-acétyl-L-cystéine.

7. Agent selon la revendication 6, **caractérisé en ce qu'**il contient en tant qu'agent réducteur, la N-acétyl-L-cystéine.

8. Agent selon l'une quelconque des revendications 1 à 7, **caractérisé en ce qu'**il contient en plus au moins un glycéride partiel d'acide gras.

9. Agent selon l'une quelconque des revendications 1 à 8, **caractérisé en ce qu'**il contient en plus au moins un épaississant.

10. Agent selon la revendication 9, **caractérisé en ce que** l'épaississant est un polymère anionique ou non ionique.

11. Agent selon l'une quelconque des revendications 9 ou 10, **caractérisé en ce qu'**il contient en tant qu'épaississant, au moins une cellulose et/ou un dérivé de cellulose.

12. Agent selon l'une quelconque des revendications 1 à 11, **caractérisé en ce qu'**il contient en plus un polymère de soin.

13. Agent selon l'une quelconque des revendications 1 à 12, **caractérisé en ce qu'**il contient au moins un tensio-actif supplémentaire.

14. Agent selon la revendication 13, **caractérisé en ce qu'**il contient un tensio-actif non ionique ou un tensio-actif amphotère.

15. Agent selon l'une quelconque des revendications 1 à 14,
**caractérisé en ce qu'**il contient au moins un agent d'alcalinisation.

16. Agent selon la revendication 15, **caractérisé en ce qu'**il contient en tant qu'agent d'alcalinisation, la monoéthanolamine, l'isopropylamine, l'isopropanolamine, le 2-amino-2-méthylpropan-1-ol, l'arginine, l'histidine, ou un hydroxyde de métal alcalin, en particulier l'hydroxyde de sodium ou l'hydroxyde de potassium.

17. Agent selon l'une quelconque des revendications 1 à 16, **caractérisé en ce qu'**il présente une valeur de pH de 4 à 10, en particulier de 6 à 9, de manière tout particulièrement préférée de 7 à 8,5.

18. Agent selon l'une quelconque des revendications 1 à 17, **caractérisé en ce qu'**il constitue une préparation de faible viscosité ayant une viscosité inférieure à 3 000 mPas (Brookfield, broche 4, 20 tr/mn, 20°C).

19. Procédé pour teindre les fibres kératiniques dans lequel un agent selon l'une quelconque des revendications 1 à 18 est appliqué sur les fibres et éliminé de nouveau par rinçage après une durée d'action.

20. Procédé pour teindre les fibres kératiniques, dans lequel est employé un agent selon l'une quelconque des revendications 1 à 18, où dans une première étape, une préparation, contenant les composants (A), (C) et (D) est appliquée sur les fibres, les fibres étant éventuellement rincées après une durée d'action, et dans une deuxième étape une préparation contenant le composant (B) est appliquée sur les fibres.

21. Utilisation d'un des agents selon les revendications 1 à 18 pour teindre les fibres kératiniques.

22. Nécessaire pour teindre les fibres kératiniques, comprenant au moins une première préparation, contenant dans un véhicule acceptable sur le plan cosmétique,
(A) au moins un produit précurseur de colorant,
(C) au moins un tensio-actif glucidique, choisi dans le groupe qui est formé par
- les alkyl- et alcényl-oligoglycosides et
- les N-alkyl-polyhydroxyalkylamides d'acide gras et
(D) au moins un agent réducteur, et une deuxième préparation, contenant dans un véhicule acceptable sur le plan cosmétique (B) au moins une enzyme qui peut catalyser l'oxydation des produits précurseurs de colorant, choisie parmi les laccases, (EC-N°1.10.3.2), les tyrosinases (EC-N°1.10.3.1), l'ascorbate oxydase (EC-N°1.10.3.3), les bilirubine oxydases (EC-N°1.3.3.5) ainsi que les phénol oxydases du type *Acremonia, Stachybotrys* ou *Pleurotus.*
